(19) Europäisches Patentamt
European Patent Office
Office européen des brevets

(11) EP 3 971 569 A1

(12) EUROPEAN PATENT APPLICATION

(43) Date of publication:
23.03.2022 Bulletin 2022/12

(21) Application number: 20306170.0

(22) Date of filing: 07.10.2020

(51) International Patent Classification (IPC):
G01N 33/68 (2006.01)        G01N 33/497 (2006.01)
G01N 33/569 (2006.01)

(52) Cooperative Patent Classification (CPC):
G01N 33/6848; G01N 33/497; G01N 33/56983

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.09.2020 FR 2009446

(71) Applicants:
• Université de Versailles
  Saint-Quentin-en-Yvelines
  78035 Versailles Cédex (FR)
• Assistance Publique-Hôpitaux de Paris
  75004 Paris (FR)
• Institut national de la santé et de la
  recherche médicale (INSERM)
  75013 Paris (FR)

• Commissariat à l'Energie Atomique et aux
  Energies
  Alternatives
  75015 Paris (FR)
• Association Hopital Foch
  92150 Suresnes (FR)

(72) Inventors:
• ROQUENCOURT, Camille
  92800 Puteaux (FR)
• THEVENOT, Etienne
  91400 Orsay (FR)
• ANNANE, Djillali
  94700 Maisons-Alfort (FR)
• GRASSIN-DELYLE, Stanislas
  92500 Rueil-Malmaison (FR)

(74) Representative: Regimbeau
20, rue de Chazelles
75847 Paris Cedex 17 (FR)

(54) **A METHOD FOR ANALYSING A SAMPLE FOR SCREENING, DIAGNOSIS OR MONITORING OF COVID-19**

(57) The method for analysis comprises:
- obtaining a sample comprising elements coming from an air expired from a person (112);
- exposing the sample to a spectrometer;
- determining values of signal intensities and/or concentrations of ions defined by their mass-to-charge ratios given by the spectrometer in a group of ranges, each range being defined by a median value and limits of the range;
- applying at least one test or decision rule to the values, the group and the test or decision rule being configured for identifying a patient suffering from COVID-19; and
- communicating a message according to a result of the test or decision rule.

FIG. 1

**Description**

The invention relates to the COVID-19 disease and to tests for screening, diagnosis or monitoring of COVID-19.
The prior art includes the following documents which are referred to in this specification:

1. COVID-19 Dashboard by the Center for Systems Science and Engineering (CSSE) at Johns Hopkins University (JHU). https://coronavirus.jhu.edu/map.html (accessed September 28th, 2020.
2. Wiersinga WJ, Rhodes A, Cheng AC, Peacock SJ, Prescott HC. Pathophysiology, Transmission, Diagnosis, and Treatment of Coronavirus Disease 2019 (COVID-19): A Review. JAMA 2020.
3. Beigel JH, Tomashek KM, Dodd LE, et al. Remdesivir for the Treatment of Covid-19 - Preliminary Report. N Engl J Med 2020.
4. Recovery_Collaborative_Group, Horby P, Lim WS, et al. Dexamethasone in Hospitalized Patients with Covid-19 - Preliminary Report. N Engl J Med 2020.
5. Lucas C, Wong P, Klein J, et al. Longitudinal analyses reveal immunological misfiring in severe COVID-19. Nature 2020.
6. Hadjadj J, Yatim N, Barnabei L, et al. Impaired type I interferon activity and inflammatory responses in severe COVID-19 patients. Science 2020.
7. Kuri-Cervantes L, Pampena MB, Meng W, et al. Comprehensive mapping of immune perturbations associated with severe COVID-19. Sci Immunol 2020; 5(49).
8. Shen B, Yi X, Sun Y, et al. Proteomic and Metabolomic Characterization of COVID-19 Patient Sera. Cell 2020; 182(1): 59-72 e15.
9. Kataoka H, Saito K, Kato H, Masuda K. Noninvasive analysis of volatile biomarkers in human emanations for health and early disease diagnosis. Bioanalysis 2013; 5(11): 1443-59.
10. Rattray NJ, Hamrang Z, Trivedi DK, Goodacre R, Fowler SJ. Taking your breath away: metabolomics breathes life in to personalized medicine. Trends Biotechnol 2014; 32(10): 538-48.
11. Amann A, de Lacy Costello B, Miekisch W, et al. The human volatilome: volatile organic compounds (VOCs) in exhaled breath, skin emanations, urine, feces and saliva. J Breath Res 2014; 8(3): 034001.
12. de Lacy Costello B, Amann A, Al-Kateb H, et al. A review of the volatiles from the healthy human body. J Breath Res 2014; 8(1): 014001.
13. Koo S, Thomas HR, Daniels SD, et al. A breath fungal secondary metabolite signature to diagnose invasive aspergillosis. Clin Infect Dis 2014; 59(12): 1733-40.
14. Nakhleh MK, Jeries R, Gharra A, et al. Detecting active pulmonary tuberculosis with a breath test using nanomaterial-based sensors. Eur Respir J 2014; 43(5): 1522-5.
15. Coronel Teixeira R, Rodriguez M, Jimenez de Romero N, et al. The potential of a portable, point-of-care electronic nose to diagnose tuberculosis. J Infect 2017; 75(5): 441-7.
16. Suarez-Cuartin G, Giner J, Merino JL, et al. Identification of Pseudomonas aeruginosa and airway bacterial colonization by an electronic nose in bronchiectasis. Respir Med 2018; 136: 111-7.
17. Schnabel R, Fijten R, Smolinska A, et al. Analysis of volatile organic compounds in exhaled breath to diagnose ventilator-associated pneumonia. Sci Rep 2015; 5: 17179.
18. Filipiak W, Beer R, Sponring A, et al. Breath analysis for in vivo detection of pathogens related to ventilator-associated pneumonia in intensive care patients: a prospective pilot study. J Breath Res 2015; 9(1): 016004.
19. Schnabel RM, Boumans ML, Smolinska A, et al. Electronic nose analysis of exhaled breath to diagnose ventilator-associated pneumonia. Respir Med 2015; 109(11): 1454-9.
20. Bos LD, Schultz MJ, Sterk PJ. Exhaled breath profiling for diagnosing acute respiratory distress syndrome. BMC Pulm Med 2014; 14: 72.
21. Bos LD, Weda H, Wang Y, et al. Exhaled breath metabolomics as a noninvasive diagnostic tool for acute respiratory distress syndrome. Eur Respir J 2014; 44(1): 188-97.
22. Schubert JK, Muller WP, Benzing A, Geiger K. Application of a new method for analysis of exhaled gas in critically ill patients. Intensive Care Med 1998; 24(5): 415-21.
23. van Geffen WH, Bruins M, Kerstjens HA. Diagnosing viral and bacterial respiratory infections in acute COPD exacerbations by an electronic nose: a pilot study. J Breath Res 2016; 10(3): 036001.
24. Traxler S, Bischoff AC, Sass R, et al. VOC breath profile in spontaneously breathing awake swine during Influenza A infection. Sci Rep 2018; 8(1): 14857.
25. Ackermann M, Verleden SE, Kuehnel M, et al. Pulmonary Vascular Endothelialitis, Thrombosis, and Angiogenesis in Covid-19. N Engl J Med 2020; 383(2): 120-8.
26. Force ADT, Ranieri VM, Rubenfeld GD, et al. Acute respiratory distress syndrome: the Berlin Definition. JAMA 2012; 307(23): 2526-33.
27. Le Gall JR, Lemeshow S, Saulnier F. A new Simplified Acute Physiology Score (SAPS II) based on a European/North American multicenter study. JAMA 1993; 270(24): 2957-63.

28. Vincent JL, Moreno R, Takala J, et al. The SOFA (Sepsis-related Organ Failure Assessment) score to describe organ dysfunction/failure. On behalf of the Working Group on Sepsis-Related Problems of the European Society of Intensive Care Medicine. Intensive Care Med 1996; 22(7): 707-10.

29. Trefz P, Pugliese G, Brock B, Schubert JK, Miekisch W. Effects of elevated oxygen levels on VOC analysis by means of PTR-ToF-MS. J Breath Res 2019; 13(4): 046004.

30. Benjamini Y, Hochberg Y. Controlling the False Discovery Rate: A Practical and Powerful Approach to Multiple Testing. J R Stat Soc Ser B 1995; 57(1): 289-300.

31. Thevenot EA, Roux A, Xu Y, Ezan E, Junot C. Analysis of the Human Adult Urinary Metabolome Variations with Age, Body Mass Index, and Gender by Implementing a Comprehensive Workflow for Univariate and OPLS Statistical Analyses. J Proteome Res 2015; 14(8): 3322-35.

32. Zou H, Hastie T. Regularization and variable selection via the elastic net. J R Stat Soc Ser B 2005; 67(2): 301-20.

33. Breiman L. Random Forests. Machine Learning 2001; 45(1): 5-32.

34. Weston J, Mukherjee S, Chapelle O, Pontil M, Poggio T, Vapnik V. Feature Selection for SVMs. Advances in Neural Information Processing Systems 13 2000.

35. Pihur V, Datta S, Datta S. RankAggreg, an R package for weighted rank aggregation. BMC Bioinformatics 2009; 10: 62.

36. Trefz P, Schmidt M, Oertel P, et al. Continuous real time breath gas monitoring in the clinical environment by proton-transfer-reaction-time-of-flight-mass spectrometry. Anal Chem 2013; 85(21): 10321-9.

37. Brock B, Kamysek S, Silz J, Trefz P, Schubert JK, Miekisch W. Monitoring of breath VOCs and electrical impedance tomography under pulmonary recruitment in mechanically ventilated patients. J Breath Res 2017; 11(1): 016005.

38. van de Kant KD, van Berkel JJ, Jobsis Q, et al. Exhaled breath profiling in diagnosing wheezy preschool children. Eur Respir J 2013; 41(1): 183-8.

39. Corradi M, Pignatti P, Manini P, et al. Comparison between exhaled and sputum oxidative stress biomarkers in chronic airway inflammation. Eur Respir J 2004; 24(6): 1011-7.

40. Rahman I. Oxidative stress, chromatin remodeling and gene transcription in inflammation and chronic lung diseases. J Biochem Mol Biol 2003; 36(1): 95-109.

These documents are referred to hereafter by their number as exponent.

As of September 28th, 2020, about 32 million of people worldwide had been infected with severe acute respiratory syndrome coronavirus 2 (SARS-CoV-2), and about 1 million had died from coronavirus disease 2019 (COVID-19).[1] Approximately 5% of patients with COVID-19 will develop acute respiratory distress syndrome (ARDS), septic shock or multiple organ dysfunction[2]. Around the world, unprecedented research efforts are being focused on the prevention, early detection, diagnosis and management of this lethal disease. To date, only one antiviral drug (remdesivir) has been approved for the treatment of patients hospitalized for COVID-19.[3] More recently, a large trial showed that dexamethasone at a daily dose of 6 mg for 10 days substantially reduced the risk of 28 day death (age-adjusted rate ratio [95% confidence interval (CI)]: 0.83 [0.75 to 0.93], particularly in patients with severe disease requiring invasive mechanical ventilation (rate ratio: 0,64 [0.51 to 0.81]).[4]

Although the early immune response may not depend on the severity of the illness, the most severely ill patients show persistent elevations of blood inflammatory markers (such as IL-1$\alpha$, IL-1$\beta$, IL-6, IL-10, IL-18 and TNF-$\alpha$) 10 days or so after SARS-CoV-2 infection, with a very high risk of subsequent organ injury.[5-7] Proteomic and metabolomic studies of serum have described a COVID-19-specific molecular signature; severe and non-severe forms of COVID-19 differ with regard to amino acid metabolism and the expression of acute phase proteins.[8]

Early diagnosis of coronavirus disease 2019 (COVID-19) is of the utmost importance but remains challenging.

An object of the invention is to contribute to the screening, diagnosis, or monitoring of this disease.

To this aim, the invention provides a method for analysis, the method comprising:

- obtaining a sample comprising elements coming from an air expired from a person;
- exposing the sample to a spectrometer;
- determining values of signal intensities and/or concentrations of ions defined by their mass-to-charge ratios given by the spectrometer in a group of ranges,

each range being defined by a median value and limits of the range;

- applying at least one test or decision rule to the values,

the group and the test or decision rule being configured for identifying a patient suffering from COVID-19; and

- communicating a message according to a result of the test or decision rule.

In one embodiment, the group comprises at least one of the following ranges, preferably all of them, and for example consists in all of them:

- m/z = 135.09 (135.03-135.17),
- m/z = 143.15 (143.07-143.22),
- m/z = 99.08 (99.04-99.14), and
- m/z = 111.12 (111.07-111.18).

In another embodiment, the group comprises at least one of the following ranges, preferably all of them, and for example consists in all of them:

- m/z = 135.09 (135.03-135.17),
- m/z = 143.15 (143.07-143.22),
- m/z = 99.08 (99.04-99.14),
- m/z = 111.12 (111.07-111.18),
- m/z = 71.05 (71.02-71.09),
- m/z = 55.05 (55.03-55.09),
- m/z = 83.09 (83.05-83.13), and
- m/z = 93.04 (93.00-93.09).

In another embodiment, the group comprises at least one of the ranges of the following table, preferably all of them, and for example consists in all of them:

| (m/z [M+H]$^+$) median | (m/z [M+H]$^+$) lower limit | (m/z [M+H]$^+$) upper limit |
|---|---|---|
| 135,09 | 135,03 | 135,17 |
| 143,15 | 143,07 | 143,22 |
| 99,08 | 99,04 | 99,14 |
| 111,12 | 111,07 | 111,18 |
| 71,05 | 71,02 | 71,08 |
| 55,05 | 55,03 | 55,09 |
| 83,09 | 83,05 | 83,13 |
| 93,04 | 93,00 | 93,09 |
| 29,01 | 29,00 | 29,03 |
| 115,11 | 115,05 | 115,18 |
| 97,07 | 97,02 | 97,10 |
| 63,01 | 62,98 | 63,04 |
| 47,05 | 47,03 | 47,08 |
| 65,06 | 65,03 | 65,10 |
| 179,15 | 179,07 | 179,25 |
| 73,07 | 73,04 | 73,11 |
| 30,98 | 30,97 | 31,00 |
| 79,06 | 79,02 | 79,10 |
| 81,07 | 81,04 | 81,12 |
| 38,03 | 38,02 | 38,06 |
| 39,02 | 39,01 | 39,04 |

(continued)

| (m/z [M+H]+) median | (m/z [M+H]+) lower limit | (m/z [M+H]+) upper limit |
|---|---|---|
| 53,04 | 53,02 | 53,07 |
| 85,10 | 85,07 | 85,15 |
| 81,02 | 80,98 | 81,06 |
| 41,00 | 40,98 | 41,03 |
| 117,09 | 117,04 | 117,16 |
| 43,99 | 43,97 | 44,02 |
| 31,00 | 30,99 | 31,02 |
| 71,09 | 71,06 | 71,13 |
| 45,03 | 45,01 | 45,06 |
| 43,97 | 43,94 | 43,99 |
| 89,06 | 89,02 | 89,11 |
| 66,96 | 66,93 | 67,00 |
| 107,09 | 107,03 | 107,15 |
| 91,06 | 91,02 | 91,11 |
| 53,00 | 52,97 | 53,03 |
| 75,05 | 75,01 | 75,09 |
| 27,00 | 26,98 | 27,01 |
| 31,02 | 31,00 | 31,03 |
| 67,06 | 67,03 | 67,09 |
| 60,05 | 60,03 | 60,09 |
| 45,00 | 44,98 | 45,02 |
| 39,03 | 39,01 | 39,05 |
| 27,01 | 26,99 | 27,03 |
| 33,03 | 33,02 | 33,05 |
| 41,04 | 41,02 | 41,06 |
| 29,00 | 28,99 | 29,02 |
| 77,06 | 77,03 | 77,10 |
| 31,05 | 31,04 | 31,07 |
| 61,04 | 61,00 | 61,07 |
| 28,00 | 27,98 | 28,01 |
| 57,03 | 57,01 | 57,06 |
| 137,13 | 137,07 | 137,21 |
| 57,07 | 57,05 | 57,10 |
| 62,03 | 62,00 | 62,06 |
| 68,06 | 68,04 | 68,10 |
| 27,02 | 27,01 | 27,04 |
| 43,06 | 43,04 | 43,08 |
| 87,08 | 87,04 | 87,13 |

(continued)

| (m/z [M+H]$^+$) median | (m/z [M+H]$^+$) lower limit | (m/z [M+H]$^+$) upper limit |
|---|---|---|
| 47,01 | 46,99 | 47,04 |
| 52,96 | 52,94 | 52,99 |
| 66,05 | 66,02 | 66,08 |
| 51,04 | 51,02 | 51,07 |
| 69,07 | 69,05 | 69,11 |
| 29,04 | 29,02 | 29,05 |

The invention relates to the metabolomics of exhaled air, among others in critically ill COVID-19 patients, and provides a COVID-19-specific breath metabolomic signature in patients requiring invasive mechanical ventilation.

This signature is formed for example by a set of 65 volatile organic compounds. Ranking aggregation showed that the eight most prominent volatile organic compounds in this signature are m/z = 135.09, m/z = 143.15, m/z = 99.08, m/z = 111.12, m/z = 71.05, m/z = 55.05, m/z = 83.09 and, m/z = 93.04, by order of importance and as defined by their mass-to-charge ratio ([M+H]$^+$) with PTR-MS detection.

Using the whole signature, we can differentiate between COVID-19 and non-COVID-19 patients with an accuracy of 93% (sensitivity: 90%, specificity: 94%, area under the receiver operating characteristic curve: 0.93-0.98).

With the eight most prominent compounds, we can differentiate between COVID-19 and non-COVID-19 patients with an accuracy of 90-93% (sensitivity: 85-90%, specificity: 93-94%, area under the receiver operating characteristic curve: 0.95-0.98).

With the four most important features (m/z = 99.08, m/z = 111.12, m/z = 135.09 and m/z = 143.15, as defined by their mass-to-charge ratio ([M+H]+) with PTR-MS detection), we can differentiate between COVID-19 and non-COVID-19 patients with an accuracy of 89-93% (sensitivity: 90-98%, specificity: 88-94%, area under the receiver operating characteristic curve: 0.89-0.95). These four most important features also discriminate the two groups (COVID-19 positive and COVID-19 negative) in the longitudinal modelling of the first 10 days of hospitalization.

Thus, non-invasive detection of volatile organic compounds in exhaled air may identify patients with COVID-19 and provide a diagnosis of COVID-19. The knowledge of this specific breathprint enables the development of rapid, non-invasive, point-of-care tests for large-scale COVID-19 screening or monitoring disease severity and control.

Thus, in one embodiment, the method is a method for the diagnosis of COVID-19.

It cannot be excluded that the invention contributes not only to the diagnosis, but also to the prognosis/evaluation of the severity and specific impairment of certain organs as well as to the evaluation of the effect of the treatments administered.

Different kinds of tests or decision rules may be used such as the algorithms known as elastic net, random forest or support vector machine, as explained below.

In one embodiment, the determining step comprises determining whether at least two, for example three, four, five, six or seven of the components are present in the sample.

In one embodiment, the spectrometer is a mass spectrometer, for example a proton transfer reaction mass spectrometer.

Using a proton transfer reaction mass spectrometer enables to have an immediate analysis of the sample and an immediate result. But other types of mass spectrometers (other than PTR-MS) would allow the detection of the same compounds.

Similarly the method may be performed according to different modalities, for example not by directly analyzing the air exhaled by the patient (thus no need for a transfer line) but by adding a storage step of the sample: the exhaled air is taken from the patient's bed and stored in bags (Tedlar bags type) or on desorption tubes (Tenax type) which are then analyzed in another place (e.g. laboratory).) with identical (PTR-MS) or similar technologies (GC-MS, GC-IMS...).

The method of the invention may also have at least one of the following features:

- the person is undergoing invasive mechanical ventilation;
- the sample is a sample of the expired air;
- the step of obtaining a sample comprises obtaining the sample via a transfer line in direct communication with the person, for example the transfer line being connected to an end of an endotracheal tube installed on the person.
- it comprises heating the transfer line; and
- the person is inspiring air, a mixture of nitrogen and oxygen or pure oxygen;

The invention also provides a device for the diagnosis, screening or monitoring of COVID-19, the device comprising:

- means for determining values of signal intensities and/or concentrations of ions defined by their mass-to-charge ratios given by a spectrometer in a group of ranges,

each range being defined by a median value and limits of the range; and

- means for applying a predetermined test or decision rule to the intensities;

the group and the test or decision rule being configured for identifying a patient suffering from COVID-19.

The device of the invention may also comprise at least one of the following features:

- means for receiving the air sample;
- a transfer line having a connector configured for connecting to an end of an endotracheal tube;
- means for heating the transfer line; and
- a spectrometer, such as a mass spectrometer, for example a proton-transfer-reaction mass spectrometer;

The invention also provides a program comprising code instructions configured for controlling an execution of the method of the invention or for controlling a device according to the invention, as well as a process for providing this program on a communication network in view of downloading the program.

Presentation of the figures

- figures 1 to 6 show graphs illustrating the results of analysis of components in the air expired from patients suffering from Covid-19;
- figure 7 shows an embodiment of a device according to the invention;
- figure 8 shows graphs obtained when performing the method of the invention with this device;
- figure 9 shows graphs obtained for the analysis of the four most prominent features (m/z = 99.08, m/z = 111.12, m/z = 135.09 and m/z = 143.15, as defined by their mass-to-charge ratio ($[M+H]^+$)) with proton transfer reaction - mass spectrometry; and
- figure 10 illustrates one of the tests or decision rules used in the embodiment of the method of the invention.

Detailed description

Breath analysis is an innovative, non-invasive, technique for detecting volatile organic compounds (VOCs) with potential for use in diagnosis and large-scale screening.[9,10] Thousands of VOCs have been identified in human breath following infectious, inflammatory or pathological events.[11,12] The analysis of exhaled air can be used to diagnose tuberculosis, invasive fungal infections, and bacterial colonization of the respiratory tract[13-16], together with ARDS and ventilator-associated pneumonia in patients in an intensive care unit (ICU).[17-22] Likewise, VOC analysis is of value in the diagnosis of viral infections in patients with chronic obstructive pulmonary disease and of influenza infections in a swine model.[23,24]

We used real-time, online, proton transfer reaction time-of-flight mass spectrometry to perform a metabolomic analysis of expired air from adults undergoing invasive mechanical ventilation in an intensive care unit due to severe COVID-19 or non-COVID-19 acute respiratory distress syndrome (ARDS).

Between March 25th and June 25th, 2020, we included 40 patients with ARDS, of whom 28 had proven COVID-19. In a multivariate analysis, we identified a characteristic breathprint for COVID-19. This signature is formed by a set of 65 volatile organic compounds. Ranking aggregation showed that the eight most prominent volatile organic compounds in this signature are m/z = 135.09, m/z = 143.15, m/z = 99.08, m/z = 111.12, m/z = 71.05, m/z = 55.05, m/z = 83.09 and, m/z = 93.04, by order of importance and as defined by their mass-to-charge ratio ($[M+H]^+$) with PTR-MS detection. Using the whole signature, we can differentiate between COVID-19 and non-COVID-19 patients with an accuracy of 93% (sensitivity: 90%, specificity: 94%, area under the receiver operating characteristic curve: 0.93-0.98). With the eight most prominent compounds, we can differentiate between COVID-19 and non-COVID-19 patients with an accuracy of 90-93% (sensitivity: 85-90%, specificity: 93-94%, area under the receiver operating characteristic curve: 0.95-0.98). With the four most important features (m/z = 99.08, m/z = 111.12, m/z = 135.09 and m/z = 143.15, as defined by their mass-to-charge ratio ($[M+H]^+$) with PTR-MS detection), we can differentiate between COVID-19 and non-COVID-19 patients with an accuracy of 89-93% (sensitivity: 90-98%, specificity: 88-94%, area under the receiver operating characteristic curve: 0.89-0.95).

The real-time, non-invasive detection of volatile organic compounds in exhaled air permits to identify patients with COVID-19.

Study design, oversight and participants

This study was conducted at the ICU of Raymond Poincaré Hospital (Garches, France). Adult patients (aged 18 or over) in ICUs were eligible for inclusion if they had ARDS and required invasive mechanical ventilation. ARDS was defined as all of the following: (i) acute onset, i.e. within one week of an apparent clinical insult, followed by progression of the respiratory syndrome, (ii) bilateral opacities on chest imaging not explained by another lung disease (e.g. pleural effusion, atelectasis, nodules etc.), (iii) no evidence of heart failure or volume overload, and (iv) $PaO_2/FiO_2 \leq 300$ mm Hg, and positive end expiratory pressure (PEEP) $\geq 5$ cm $H_2O$.[26] The main exclusion criteria were pregnancy, an expectation of death within 48 hours, and the withholding or withdrawal of treatment.

Study measurements and procedures

Variables recorded at baseline were patient demographics and anthropometrics, the source of infection and the severity of illness (according to the Simplified Acute Physiology Score (SAPS) II and the Sequential Organ Failure Assessment (SOFA)).[27,28] The following variables were recorded at baseline and daily during the hospital stay: core body temperature, vital signs, central hemodynamic data, standard laboratory data, microbiological and virologic data. Samples of blood and nasopharyngeal, bronchial or bronchoalveolar lavage fluids were assayed for SARS-CoV-2 and other respiratory viruses, using a PCR test. Were also recorded life-supportive therapies including mechanical ventilation, renal replacement therapy, intravenous fluids bolus and the administration of vasopressors, and adjunct therapies including corticosteroids, thiamine, vitamin C, other vitamins, nutritional supplements, blood products, anticoagulants, sedatives, stress ulcer prophylaxis and anti-infective drugs.

Breath analysis

Each patient's expired air was analyzed daily in the morning until discharge. Measurements were made with a proton-transfer-reaction quadrupole time-of-flight mass spectrometer (PTR-Qi-TOF MS) (Ionicon Analytik GmbH, Innsbruck, Austria) placed outside the patient room. Samples were obtained via a heated transfer line (length: 1,6 m) connected directly to the end of the endotracheal tube (i.e. without disconnection from the mechanical ventilator) and with an air flow of 50 mL/min. To eliminate the dependency on the oxygen concentration in the sample matrix, recordings were performed in patients with a fraction of inspired oxygen of 100% for at least 3 mins.[29] The acquisition took 2 mins. $H_3O^+$ was used as the primary ion and the instrument settings were as follows: source voltage, 120 V; drift tube pressure, 3.8 mbar; drift tube temperature, 60° C; and drift tube voltage, 959 V. The mass spectrum was acquired up to m/z = 392, with a time resolution of 0.1 s.

We recall that, when presenting data of a spectrometer, it is common to use the (officially) dimensionless m/z. This quantity, although it is informally called the mass-to-charge ratio, more accurately speaking represents the ratio of the mass number m and the charge number, z. A spectrometer can measure this mass-to-charge ratio of ionized compounds. For example for a molecule M, the form $[M+H]^+$ may be detected with PTR-MS when using $H_3O^+$ as the primary ion.

Data and statistical analysis

Patient characteristics were expressed as the median [interquartile range (IQR)] for continuous variables and the frequency (percentage) for categorical variables. Patients with and without COVID-19 were compared using Fisher's exact test for categorical variables, and a t-test or the Mann-Whitney test for normally and non-normally distributed continuous variables (as evaluated with the d'Agostino-Pearson test), respectively.

Mass spectrometry data were processed with the *ptairMS* software developed by CEA under the CeCILL licence (https://github.com/camilleroquencourt/ptairMS) and included mass calibration, expiratory phase detection, peak detection and quantification, normalization, alignment, isotope identification and the imputation of missing values. After aligning each individual peak, ions detected in more than 70% of at least one group (COVID vs. non-COVID-19) were kept; this resulted in 81 features. Missing values (corresponding to ions in exhaled air that were not detected by the preprocessing algorithm) were imputed with the *ptairMS* package, which returns to the raw data and integrates the noise at the exact missing m/z. Data were then log2-transformed and standardized. Outliers (patients with a z-score >3 for at least five features) were deleted. In the remaining patients, saturated ions (acetone, $H_3O^+$, $H_2O$-$H_3O^+$, oxygen) and isotopes were deleted to leave a final table of 65 features.

For the univariate analysis, a Wilcoxon test was performed and p-values were adjusted to control for the false discovery rate.[30] For multivariate analysis, data were first analyzed with unsupervised learning principal component analysis and then with supervised machine learning algorithms (orthogonal partial least-squares discriminant analysis, linear support vector machine, elastic net, and random forest) with the R packages *ropls, e1071, glmnet,* and *random-Forest*.[31-34] A 10-fold cross-validation was repeated four times, in order to avoid overfitting the small number of data points. The model's parameters were tuned to optimize the cross-validation accuracy.

To rank the features in order of importance, we used rank aggregation (with *RankAggreg* R package[35]) of the ordered p-values from the Wilcoxon test, the rank in decreasing order of absolute value for the loadings in the principal component analysis, the variable importance in projection from the orthogonal partial least-squares discriminant analysis, the coefficient values from the elastic net and the support vector machine, and the feature importance from the random forest. The effects of tidal volume, PEEP, respiratory rate, serum C-reactive protein (CRP) level, body temperature, and renal replacement were also investigated in a correlation test; the secondary principal component in the principal component analysis was used to determine whether or not these covariates were confounding factors (using Pearson's test for continuous variables and a chi-squared test for categorical variables).

Longitudinal analysis of the most important features was performed with a mixed effects model, using a sum of 4 spline functions uniformly distributed over time for the fixed effect. Intergroup differences in trends and mean concentrations were assessed with an F-test (p-value <0.05) adjusted to correct for the false discovery rate. Correlations between VOC concentrations, the SAPS II, the SOFA score and the viral load were analyzed using Pearson's correlation test, with correction for the false discovery rate.

Results

Patients

Between March 25th and June 25th, 2020, 40 patients (of whom 28 had confirmed COVID-19-related ARDS) were included in the study and a total of 303 measurements were made.

Compared with the patients with non-COVID-19 ARDS, the patients with COVID-19 ARDS had (i) a higher incidence of treatment with glucocorticoids prior to admission, (ii) a higher respiratory rate, $FiO_2$, PEEP and CRP on admission, (iii) a higher incidence of treatment with hydroxychloroquine and fludrocortisone after admission, and (iv) a greater likelihood of renal replacement therapy (Table 1).

Table 1: Patient characteristics and treatments

| | COVID-19 ARDS | Non-COVI D-19 ARDS | p value |
|---|---|---|---|
| Number of patients (n) | 28 | 12 | - |
| Males/females (n) | 20/8 | 6/6 | 0.28 |
| Age (years) | 61 [55-72] | 72 [54-79] | 0.75 |
| Body weight (kg) | 80.0 [66.6-87.6] | 86.5 [65.3-94.1] | 0.71 |
| Height (cm) | 170 [164-175] | 173 [169-175] | 0.55 |
| Body mass index ($kg/m^2$) | 26.3 [23.7-32.4] | 28.9 [23.0-30.9] | 0.79 |
| SAPS II score in the first 24 hours | 62 [49-68] | 46 [40-57] | 0.051 |
| SOFA score in the first 24 hours | 11 [7-12] | 8 [5-12] | 0.37 |
| Comorbidities: (n (%))<br> - high blood pressure<br> - chronic obstructive pulmonary disease<br> - ischemic cardiac disease<br> - cancer | <br>11 (39)<br>2 (7)<br>5 (18)<br>2 (7) | <br>6 (50)<br>1 (8)<br>3 (25)<br>3 (25) | <br>0.73<br>>0.99<br>0.68<br>0.15 |
| Treatments before admission: (n (%))<br> - glucocorticoids<br> - conversion enzyme inhibitors<br> - angiotensin antagonists | <br>0 (0)<br>5 (18)<br>2 (7) | <br>3 (25)<br>1 (8)<br>2 (16) | <br>0.022<br>0.54<br>0.57 |
| Interventions after admission: (n (%)) | | | |

(continued)

| | COVID-19 ARDS | Non-COVI D-19 ARDS | p value |
|---|---|---|---|
| - catecholamines | 17 (61) | 4 (33) | 0.17 |
| - renal replacement therapy | 9 (32) | 0 (0) | 0.038 |
| Treatments after admission: (n (%)) | | | |
| - hydroxychloroquine | 27 (96) | 1 (8) | <0.0001 |
| - remdesivir | 2 (7) | 0 (0) | >0.99 |
| - lopinavir/ritonavir | 7 (25) | 0 (0) | 0.081 |
| - glucocorticoids | 11 (39) | 6 (50) | 0.73 |
| - fludrocortisone | 1 (4) | 4 (33) | 0.022 |
| - eculizumab | 12 (43) | 4 (33) | 0.73 |
| Body temperature at first sample (°C) | 37.4 [36.5-38.3] | 37.3 [36.8-37.8] | 0.84 |
| Respiratory rate at first sample (breaths per min) | 26 [25-28] | 20 [18-23] | <0.0001 |
| Tidal volume at first sample (mL) | 420 [400-475] | 438 [400-490] | 0.99 |
| Fraction of inspired oxygen at first sample (%) | 80 [50-100] | 48 [31-68] | 0.007 |
| Positive end-expiratory pressure at first sample (cm $H_2O$) | 10 [8-13] | 5.5 [5-8] | 0.0002 |
| Serum creatinine at first sample ($\mu$M) | 74 [56-137] | 67 [44-86] | 0.30 |
| Serum C-reactive protein at first sample (mg/L) | 195 [175-268] | 76 [23-119] | 0.002 |
| Continuous data are presented as the median [IQR]. | | | |

Metabolomic analysis of exhaled air

We first used an untargeted metabolomic strategy to identify biomarkers associated with COVID-19 ARDS. To this end, we used the first breath sample collected after admission. Twelve of the 40 participants had been hospitalized for more than 10 days at the start of the sampling period and so were excluded from this first part of the study. Hence, we analyzed 18 patients with COVID-19 ARDS and 10 with non-COVID-19 ARDS.

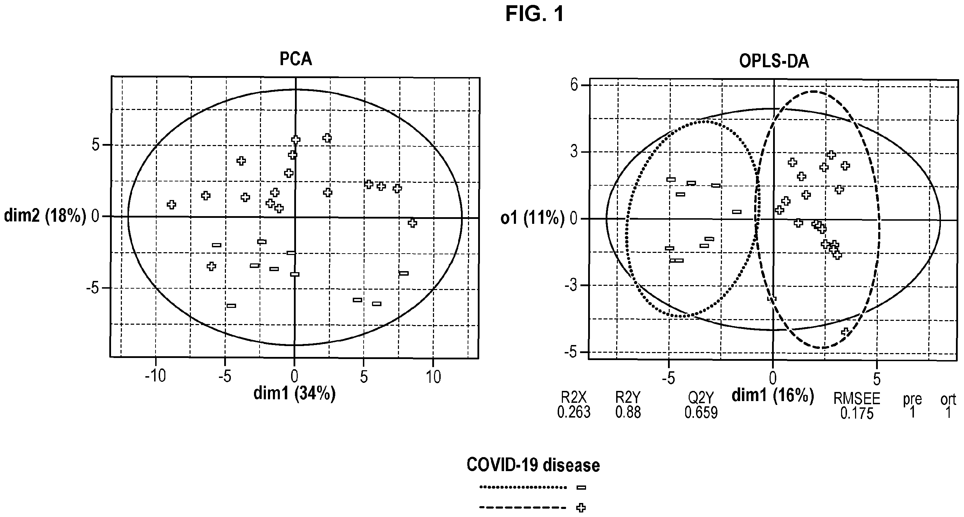

A principal component analysis and an orthogonal partial least-squares discriminant analysis showed that COVID-19 was associated with a specific signature in the expired air, i.e. the breathprint could discriminate between COVID-19 and non-COVID-19 patients. Figure 1 shows this unsupervised analysis. A primary component analysis (left) and an orthogonal primary least-squares discriminant analysis (right) of the breath signature in intubated, mechanically ventilated ICU patients with a positive (red) or negative (blue) PCR test for SARS-CoV-2.

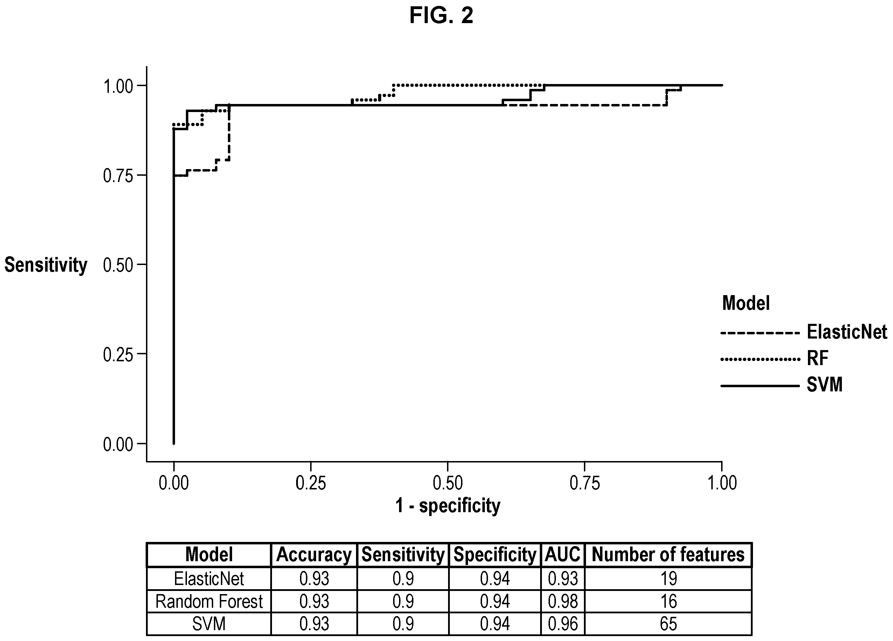

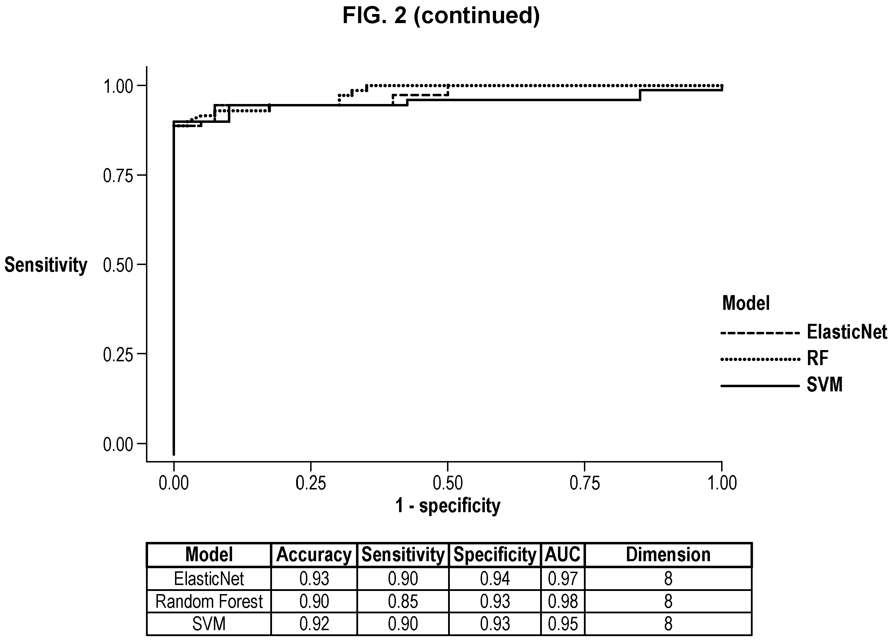

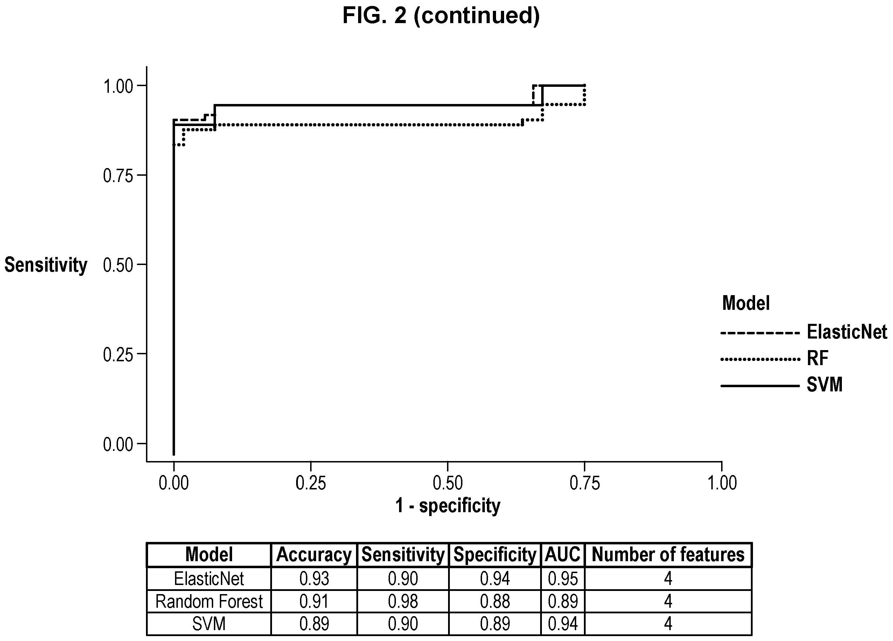

The use of three machine learning classification algorithms (elastic net, support vector machine and random forest) yielded an accuracy of up to 93%, with a 10-fold cross validation repeated 4 times, using 19 (elastic net), 16 (random forest) or 65 (support vector machine) features from the 65 features of the whole signature. The corresponding receiver operating characteristic curves are shown in Fig. 2a. These curves are for models classifying patients with COVID-19 vs. non-COVID-19 ARDS. After internal cross-validation, the sensitivity was 90% and the specificity was 94%.

In order to identify possible confounding effects, we first tested the relationship between the COVID-19 status and all the available covariables for patient demographics (sex, age, body weight, height, and body mass index), clinical and biological data (body temperature, SAPS II and SOFA scores, serum CRP and serum creatinine), comorbidities (high blood pressure, chronic obstructive pulmonary disease, ischemic cardiac disease and cancer), ventilation parameters (respiratory rate, positive end-expiratory pressure and tidal volume) and treatments (catecholamines, renal replacement, hydroxychloroquine, remdesivir, lopinavir/ritonavir, glucocorticoids, fludrocortisone and eculizumab). We applied a Wilcoxon test to quantitative variables and a chi-squared test to qualitative variables, with false discovery rate correction in both cases.

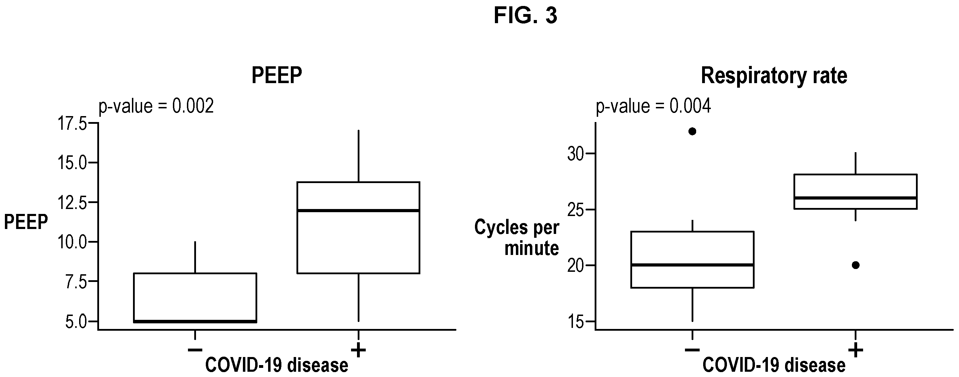

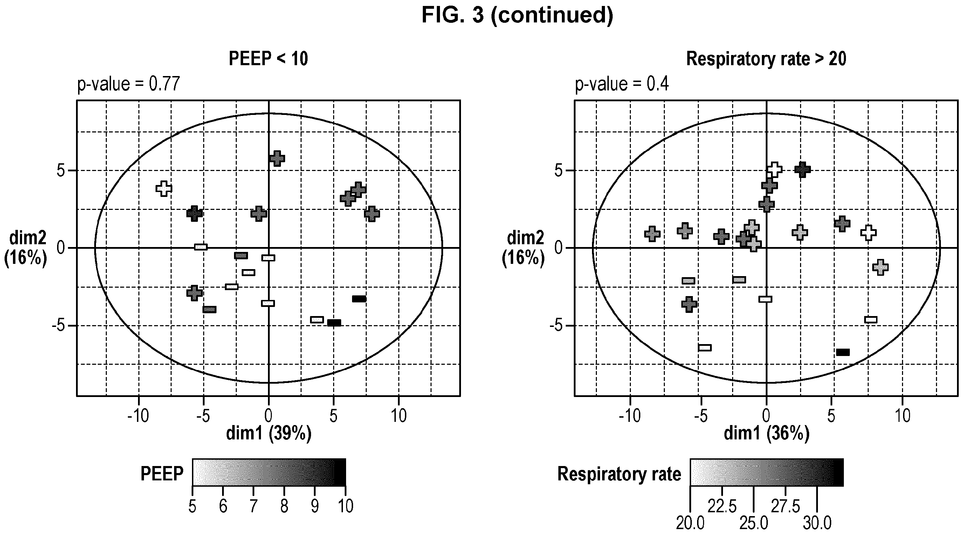

The variables significantly related to COVID-19 status (p-value <0.05) were the positive end-expiratory pressure and the respiratory rate (Fig. 3 - A). Figure 3 shows the effect of the positive end-expiratory pressure and respiratory rate as follows:

A. The positive end-expiratory pressure (PEEP) and respiratory rate, according to COVID-19 ARDS status. The p-value from the Wilcoxon test is indicated.

B. A principal component analysis of the subsets corresponding to patients with PEEP < 10 (left) or a respiratory rate > 20 (right).

The "+" and "-" symbols refer to the patient's COVID-19 status. The p-value from the Pearson correlation test comparing the factor intensities (displayed as a color gradient) and the scores in the secondary component of the principal component analysis (i.e. the component that best discriminated between COVID-19 ARDs and non-COVID-19 ARDS) is shown.

To determine whether these variables had a confounding effect, we therefore restricted the sample to two rebalanced groups by keeping only patients with a positive end-expiratory pressure <10 on one hand or a respiratory rate >20 on the other. We then applied Pearson's correlation test to the secondary component from the principal component analysis of this restricted dataset. We observed that in both cases the secondary component still distinguished between cases of COVID-19 ARDS and non-COVID-19 ARDS disease and that there was no correlation with the positive end-expiratory pressure or the respiratory rate (Fig. 3 - B).

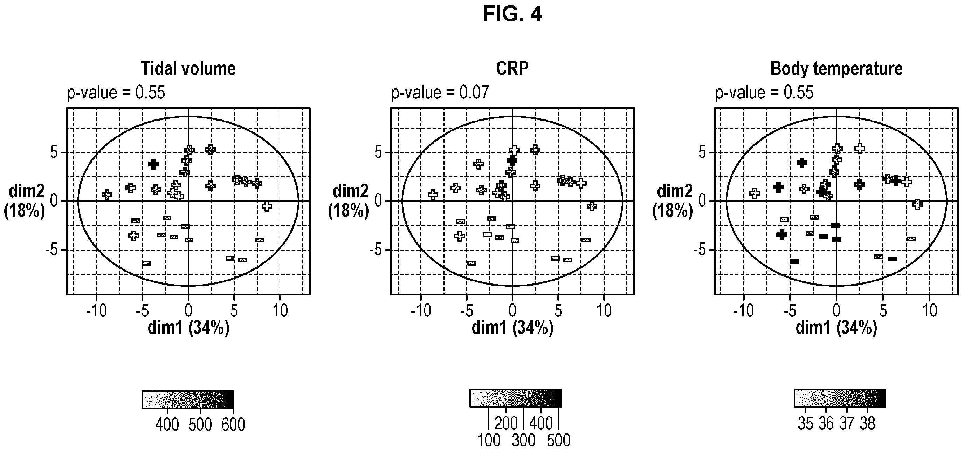

We used the same procedure for the other covariables with the complete dataset. None of them was significantly correlated with the secondary component of the principal component analysis (Fig. 4). Figure 4 shows the effect of tidal volume, CRP and body temperature in a principal component analysis and a correlation analysis. COVID-19 status is represented by the "+" and "-", whereas the color scale represents the factor's intensity. The p-value of the Pearson correlation test comparing the secondary component and the corresponding factor is shown on each graph.

To determine which VOCs were most discriminant for COVID-19 status, we performed rank aggregation using the various metrics from the previously mentioned models and the hypothesis tests.

The eight most prominent volatile organic compounds in this signature were m/z = 135.09, m/z = 143.15, m/z = 99.08, m/z = 111.12, m/z = 71.05, m/z = 55.05, m/z = 83.09 and, m/z = 93.04, by order of importance and as defined by their mass-to-charge ratio ([M+H]+) with PTR-MS detection. With these eight most prominent compounds, we can differentiate between COVID-19 and non-COVID-19 patients with an accuracy of 90-93% (sensitivity: 85-90%, specificity: 93-94%, area under the receiver operating characteristic curve: 0.95-0.98).

With the four most important features (m/z = 99.08, m/z = 111.12, m/z = 135.09 and m/z = 143.15, as defined by their mass-to-charge ratio ([M+H]+) with PTR-MS detection), we can differentiate between COVID-19 and non-COVID-19 patients with an accuracy of 89-93% (sensitivity: 90-98%, specificity: 88-94%, area under the receiver operating characteristic curve: 0.89-0.95).

The corresponding receiver operating characteristic curves are shown in Fig. 2b for the eight most prominent compounds (m/z = 135.09, m/z = 143.15, m/z = 99.08, m/z = 111.12, m/z = 71.05, m/z = 55.05, m/z = 83.09 and, m/z = 93.04) and Fig. 2c for the four most prominent compounds (m/z = 135.09, m/z = 143.15, m/z = 99.08 and m/z = 111.12).

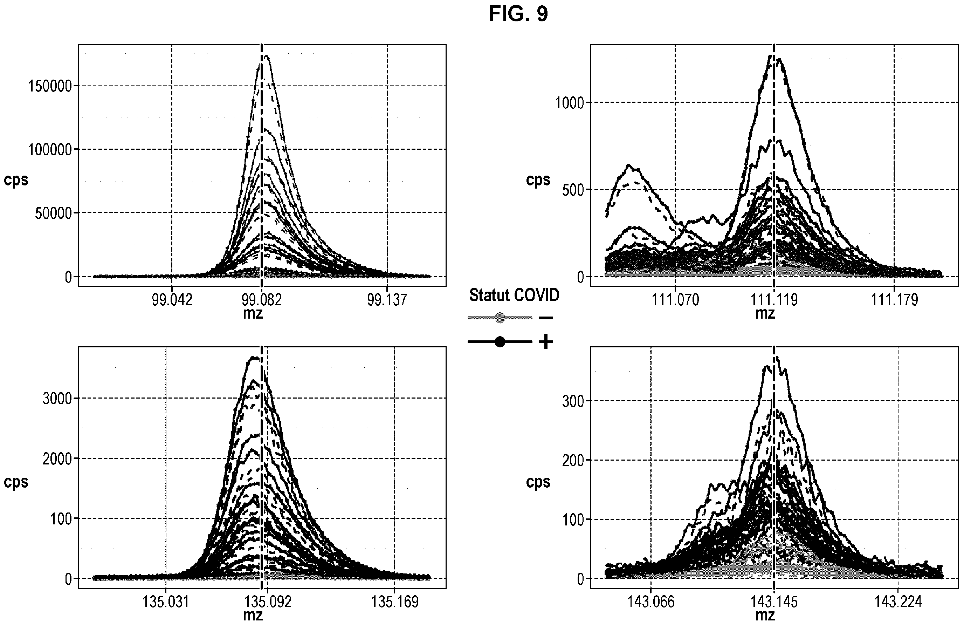

Figure 9 shows graphs obtained for the analysis of the four most prominent features (m/z = 99.08, m/z = 111.12, m/z = 135.09 and m/z = 143.15, as defined by their mass-to-charge ratio ([M+H]$^+$)) with proton transfer reaction - mass spectrometry. Each curve represents a patient of the study. And the curves of the patients having a COVID+ status and a COVID- status are visually differentiated. The range associated with each median value is illustrated.

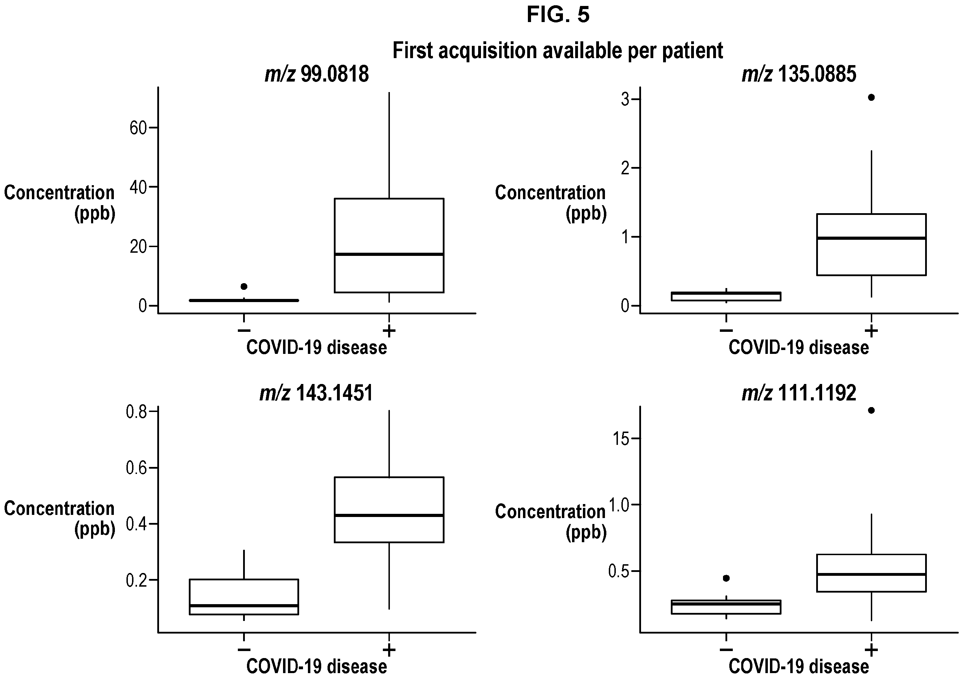

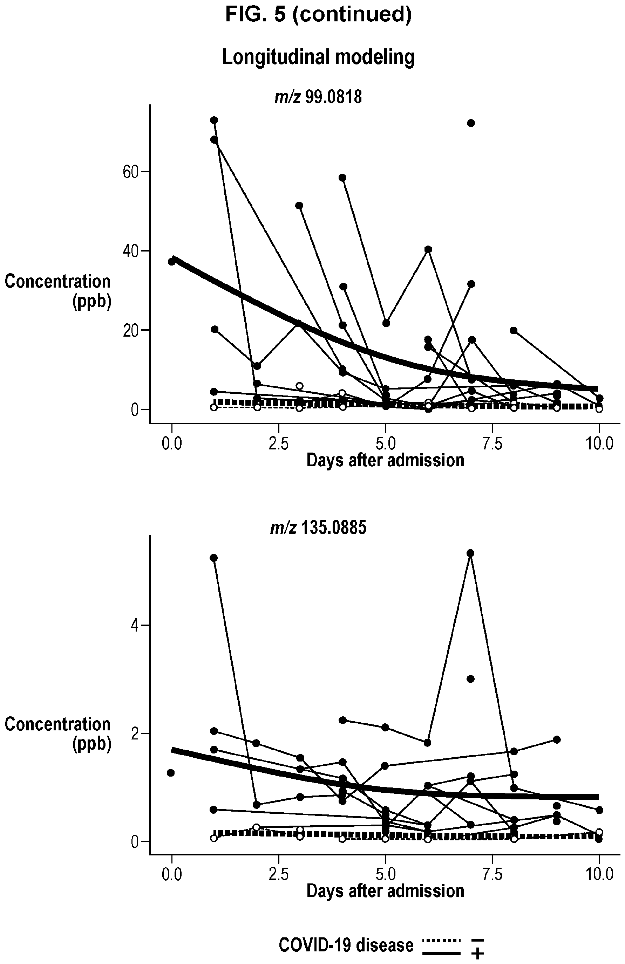

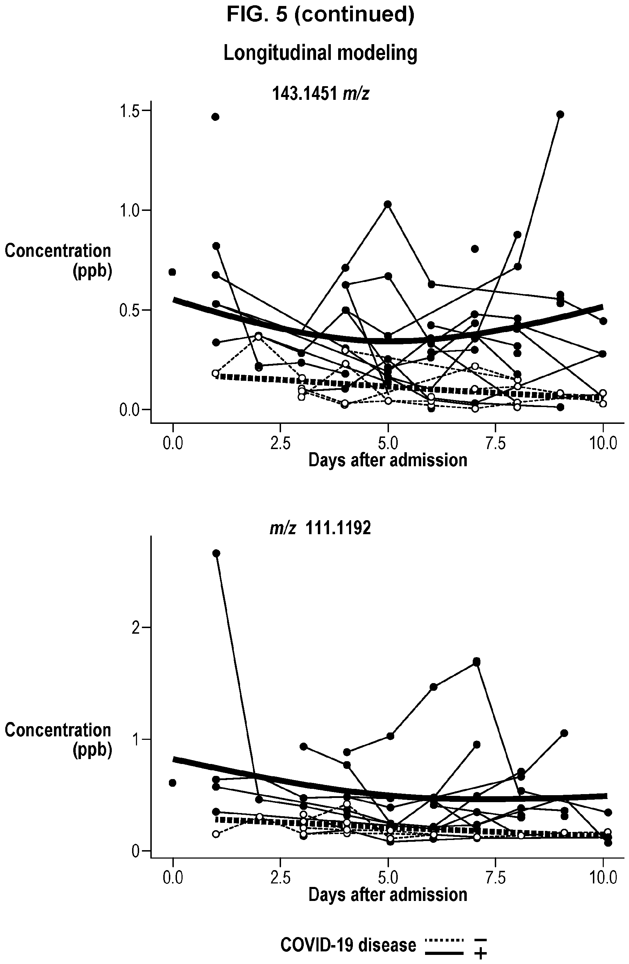

We investigated the expression of the four most prominent VOCs in the whole study population throughout the period of mechanical ventilation (Fig. 5b). Figure 5 shows the longitudinal analysis of VOCs in expired air. The four features (m/z 99.08, 135.09, 143.15 and 111.12) contributing the most to the models were assessed in the first sample available for each patient (a) and over time (b) during the ICU stay of intubated, mechanically ventilated patients with COVID-19 (in red) or non- COVID-19 ARDS (in blue). All the points for a given patient are connected, and the bold lines correspond to the fixed effect of the mixed model for each group.

We observed that the VOC concentrations (i) were significantly higher in the breath of patients with COVID-19 ARDS than in the breath of patients with non-COVID-19 ARDS, and (ii) tended to decrease over the first 10 days of hospitalization. The putative annotations for the four compounds at m/z 135.09, 143.15, 99.08 and 111.12 were respectively 1-chloroheptane, nonanal, methylpent-2-enal, and 2,4-octadiene.

Correlation with viral load and severity scores

The viral load in bronchoalveolar fluid was measured for 18 patients. The median [IQR] value in the first sample was 7.2 [6.2-8.4] log eq. copies/mL. The VOC concentrations in the first sample were not significantly correlated with the bronchoalveolar fluid viral load or with the severity of illness (i.e. the SAPS II and SOFA score[27,28]) measured during the first 24 hours in the ICU (Table 2).

Table 2: Absence of significant correlations between VOC concentrations and the SAPS II, SOFA score and viral load.

| VOC (m/z) | SAPS II score | | SOFA score | | Viral load | |
|---|---|---|---|---|---|---|
| | *r* | *p*-value | *r* | *p*-value | *r* | *p*-value |
| 143.15 | 0.12 | 0.62 | 0.27 | 0.25 | -0.23 | 0.24 |
| 135.09 | 0.05 | 0.85 | 0.35 | 0.14 | -0.0004 | 1.00 |
| 99.08 | 0.04 | 0.88 | 0.36 | 0.13 | 0.08 | 0.70 |
| 111.12 | 0.02 | 0.93 | 0.28 | 0.25 | -0.14 | 0.48 |
| r: Pearson's correlation coefficient. | | | | | | |

This study provided proof of concept for the measurement of VOCs and the determination of a specific VOC breathprint in the exhaled air from patients with COVID-19-related ARDS requiring invasive mechanical ventilation in the ICU. This breathprint was independent of the severity of illness and the viral load. Four features (m/z 99.08, 111.12, 135.09 and 143.15) discriminate between COVID-19 and non-COVID-19 ARDS in the longitudinal analysis.

Two of these four prominent VOCs (m/z = 143.15 and m/z = 99.08, which may correspond to methylpent-2-enal and nonanal) are aldehydes, while 2,4-octadiene (m/z = 111.12) is an alkadiene; all three compounds are known to be expressed in breath.[38,39] Nonanal (m/z = 99.08) is a sub-product of the destruction of the cell membrane as a result of oxidative stress; reactive oxygen species may be generated by various type of inflammatory, immune and structural cell in the airways.[40] In studies of expired air from patients with ARDS, Schubert *et al.* found abnormally low isoprene concentrations and Bos *et al.* reported abnormally high concentrations of octane, acetaldehyde and 3-methylheptane.[21,22] Differences in study populations (non-COVID-19 *vs.* COVID-19 ARDS) and analytical methods (offline *vs.* online) might explain the differences between the VOCs identified in the present study and those identified in previous studies of ARDS.[21,22]

In line with previous reports, the VOC concentrations measured here were not correlated with the severity of illness (as judged by the SAPS II and the SOFA score).[21] This finding suggests that the exhaled air signature is a marker of COVID-19 *per se,* rather than of the severity of illness. Likewise, the VOC concentrations were not correlated with viral load, suggesting that this signature may be a marker of the disease related to SARS-CoV-2 rather than of virus carriage.

Our interpretation of the present data may have been limited by differences between the COVID-19 and non-COVID-19 ARDS subgroups. Patients with COVID-19 ARDS cohort had higher respiratory rate, $FiO_2$, PEEP and CRP values on admission. The respiratory rate, PEEP and CRP were not found to be confounding factors, and all the patients were sampled when breathing 100% $FiO_2$ (to avoid mass spectrometry interference by oxygen).[29]

The FiO2 represents the percentage of oxygen in the air with which patients are ventilated, the values range from 21% (ambient air) to 100% (pure oxygen, for the most severe patients). This percentage of oxygen causes interference for analysis with a PTR-MS. For this reason, for all patients, we first performed a first analysis with their basal $FiO_2$ level (between 21 and 100%), then all patients were ventilated with 100% $FiO_2$ for 5 mins to have a standardized value and a second analysis was performed. Only the results of this second analysis are detailed here.

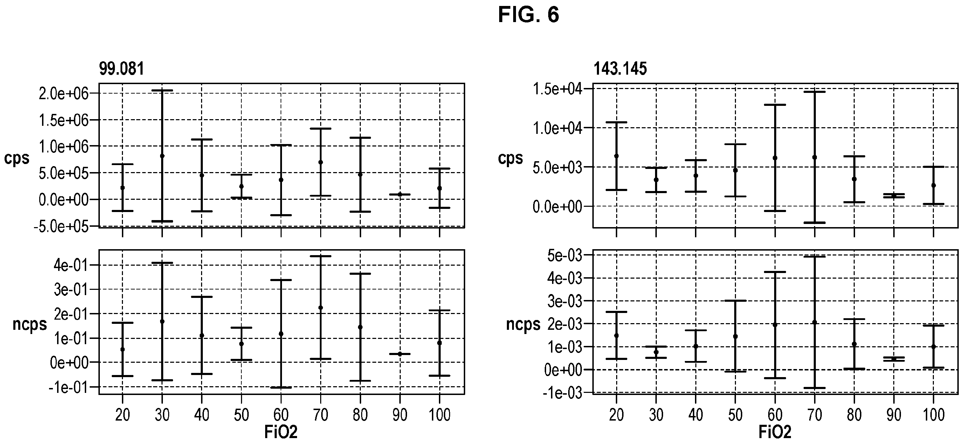

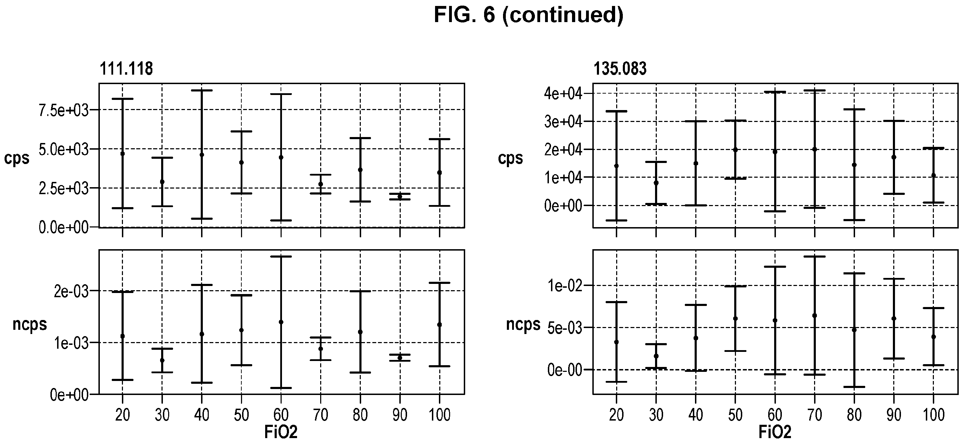

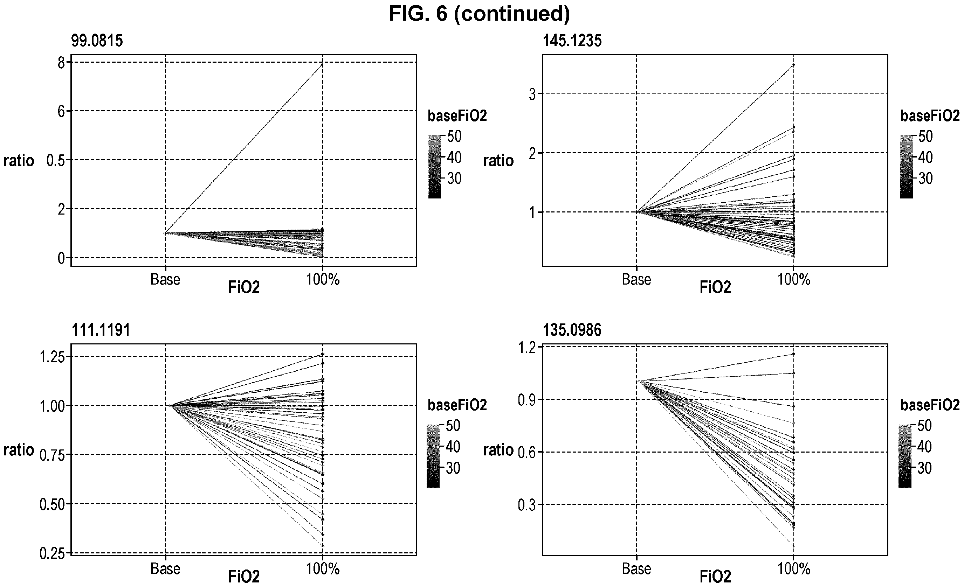

The graphs of figure 6 represent, for each of the four compounds of interest, the values measured in the two analyses. The first four graphs show the values obtained for all patients as a function of the initial % $FiO_2$ ("raw" values (cps, upper line), or normalized (ncps, lower line, normalization process not detailed here). The four other graphs of the figure represent the variations observed for each patient between the 1st measurement (value on the left, "base") and the 2nd measurement in 100% $FiO_2$ (value on the right). The statistical analyses performed do not show any significant difference, which suggests that the percentage of oxygen does not disturb the analysis of the 4 compounds of interest.

Similarly, patients with COVID-19 ARDS were more likely to have been treated with hydroxychloroquine and fludrocortisone. However, these drugs were administered to the patients after their first sample had been analyzed. Although the VOC concentrations decreased over time, the treatments did not change. Lastly, there is no correspondence between VOCs described in the present study and the molecular masses of the known metabolites of hydroxychloroquine or fludrocortisone.

The *ptairMS* R package used for data analysis is fully available at https://github.com/camilleroquencourt/ptairMS.

Due to these findings, we can apply a highly sensitive, rapid, non-invasive, real-time mass spectrometry breath analysis.[36,37] This contrasts with offline technologies, which require a sampling step and remote, time-consuming analytical steps.[21,22] The subsequent diagnostic validation and clinical implementation can be based on less cumbersome technologies, such as mass spectrometers dedicated to targeted analyses or portable "electronic noses" with a set of sensors that are relatively selective for different families of VOCs (as previously used in patients with ARDS).[20]

Embodiment

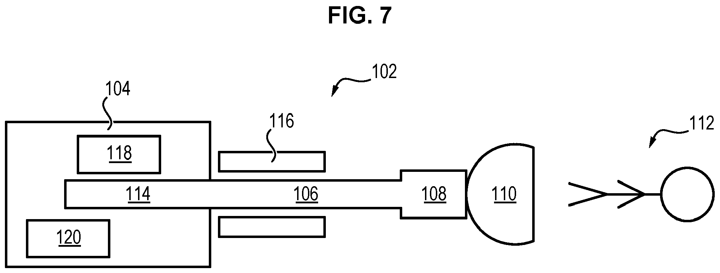

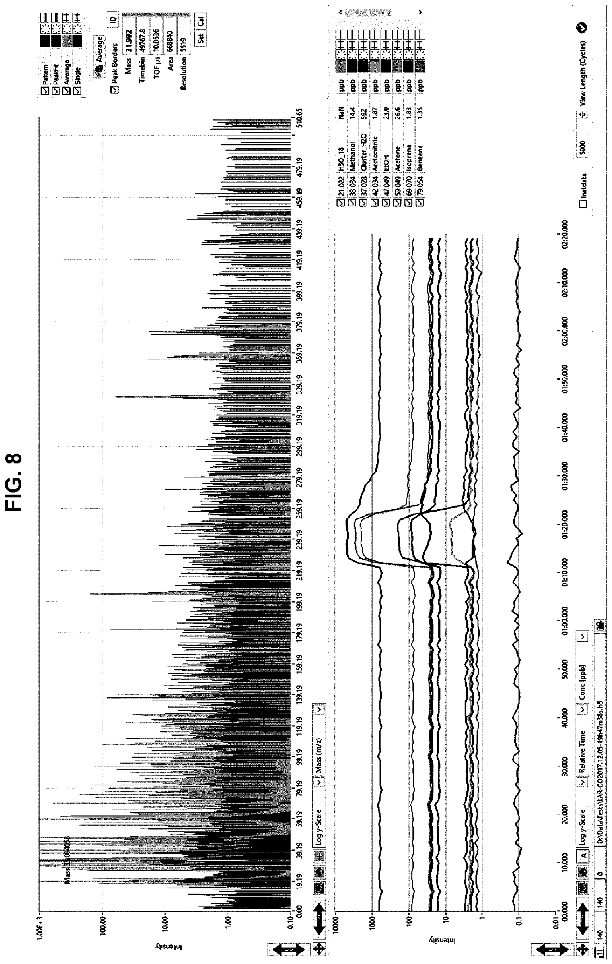

We will now present a first embodiment of the device for screening, diagnosis or monitoring of COVID-19, with reference to figures 7 and 8.

The device 102 comprises a body 104.

The device comprises means for receiving an air sample. These means comprise an elongated transfer line 106. One end of the transfer line has a connector 108 configured for connecting to an end of an endotracheal tube 110. This tube is configured for installation on the face of a person 112 for invasive mechanical ventilation. Another end 114 of the transfer line 106 extends inside body 104.

The device also comprises means 116 for heating the transfer line, which helps to preserve the components or compounds from the moment they exit the patient to the moment the air sample is analyzed inside device 102.

The device comprises inside body 104 means configured for determining whether m/z = 135.09, m/z = 143.15, m/z = 99.08 and m/z = 111.12 are present in an air sample. These means comprise in this example a proton-transfer-reaction mass spectrometer 118 configured for analyzing an air sample transferred through line 106.

The device has computer and/or electronic control means 120. These means comprise a program comprising code instructions configured for controlling device 102 for performing the method presented hereafter. This program can be provided on a communication network in view of downloading the program into the device when it is connected to this network with classical means in this view.

The method of the invention is performed as follows with this device 102.

We assume that the person 112 is undergoing invasive mechanical ventilation. An endotracheal tube 110 is installed on the person. The connector 108 of transfer line 106 is connected to the endotracheal tube 110. The person is breathing for example air of 100% $FiO_2$, which means that this person inspires pure oxygen.

The method comprises the step of heating the transfer line with the heating means 116.

It then comprises the step of obtaining a sample of expired air from the person 112 via the heated transfer line. Thus, the sample of expired air enters body 104 for being exposed to the spectrometer and analyzed.

The method then comprises the step of determining whether m/z = 135.09, m/z = 143.15, m/z = 99.08 and m/z = 111.12 are present in the sample.

To this end, the spectrometer 118 and the control means 120 perform the steps of determining values of signal intensities and/or concentrations of ions defined by their mass-to-charge ratios (m/z) given by the spectrometer in a group of ranges, each range being defined by a median value and limits of the range. In this example, the group consists of the following ranges:

- m/z = 135.09 (135.03-135.17),
- m/z = 143.15 (143.07-143.22),
- m/z = 99.08 (99.04-99.14),
- m/z = 111.12 (111.07-111.18).

Figure 8 shows on the second graph the amount of certain components into the air received inside the device as a function of time. The sample is received inside the device roughly between 1 min 10 s and 1 min 20 s. At that moment, the amount of some components rises and then decreases.

The first graph shows the intensity or concentration of different components as a function of their mass (m/z). Each peak thus designates a different component. The higher the peak, the higher the intensity or concentration of the component in the expired air. The device can directly and in real time evaluate whether one, two, three or all of the eight components are present in the expired air.

For this purpose, the device determines the value m/z of each of the components ($[M+H]^+$) in the sample.

Then we use these signal intensities or concentrations to predict the probability of COVID infection thanks to a machine learning model trained with the data. For that, the method comprises the step of applying at least one test or decision rule to the values, the group and the test or decision rule being configured for identifying a patient with COVID-19.

In this embodiment, the tests or decision rules of elastic net, random forest and support vector machine (SVM) can be used for example. This testing step is performed as follows for example.

We log-transform the measured concentration in ppb of k VOCs of interest. We note x = $(x_1,..., x_k)$ those values.

Elastic net[32]

We generally define the probability that this individual is in the COVID-19 positive group as follows:

$$p = logit^{-1}\left(\alpha_0 + \sum_{i=1}^{k} \alpha_i \times x_i\right)$$

**where** $(\alpha_0,..., \alpha_k)$ are known coefficient estimated from our data, and

$$logit^{-1}(x) = \frac{\exp(x)}{1 + \exp(x)}$$

If p is greater than a certain threshold (e.g. 0.5), the individual is considered to be in the COVID-19 positive group.

In our example with the four VOC of interest, we note $x_{99}, x_{111}, x_{135}, x_{143}$ the logarithm of ions 99.08, 111.12, 135.09 and 143.15 concentrations.

After training and tuning the model, we obtain the decision rules:

$$p = logit^{-1}(4.32692 + 0.0717 \times x_{99} - 0.88062 \times x_{111} + 1.51188 \times x_{135} + 1.82797 \times x_{143})$$

If p is greater than 0.5, the individual is considered to be in the COVID-19 positive group.

Random forest

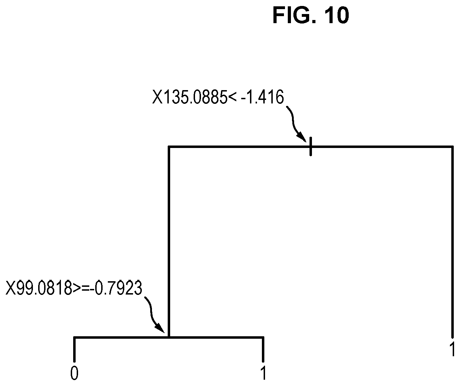

The Random Forest approach is based on the aggregation of the predictions from many decision trees[33]. Here, five hundred decision trees as the one illustrated on figure 10 have been built from our data. Each tree is built from a random subset of our variables. For the illustrated tree, if the logarithm of the ions 135.09 is less than -1.416, the patient is considered to belong to the positive group. Else, we look at the log-concentration of the ion 99.09. If this is less than - 0.7923, the patient is in the negative group. We note the result for the five hundred trees, which are negative (0) or positive (1). If there is more positive outputs than negative outputs, the individual is considered to be in the COVID-19 positive group.

SVM[34]

An equation of a hyperplane h(x)=0 separating the two classes has been estimated from our data, for example in the linear case:

$$h(x) = \sum_{i}^{k} \alpha_k x_k + \alpha_0$$

or in general case:

$$h(x) = \sum_{j}^{p} \alpha_j K(x^j, x) + \alpha_0$$

where $(x^1,..x^p)$ are the features value for patient used to build the hyperplane (called support vector), K a kernel and $(\alpha_i)$ the known estimated coefficients.

If h(x)>0 (respectively h(x)≤0), the patient is in the COVID-19 positive (respectively negative) group.

In our examples with the four VOC of interest, we note $x_{99}$, $x_{111}$, $x_{135}$, $x_{143}$ the logarithm of ions m/z = 99.08, 111.12, 135.09, 143.15 concentration, whose mean and variance have been scaled according to the values from the training data..

After training and tuning the model, we obtain the decision rule:

$$h(x_{99}, x_{111}, x_{135}, x_{143}) = 0.8776471 + 1.1364761\, x_{135} + 0.8867700\, x_{143} + 0.3212751\, x_{99} -$$

$$0.1639539\, x_{111}$$

If $h(x_{99}, x_{111}, x_{135}, x_{143}) > 0$, the patient is in the COVID-19 positive group.

One or at least two of these tests or decision rules are performed.

Then a message is communicated according to the result of the test(s) or decision rule(s). This comprises for example displaying the result of the test or decision rule on a screen of the device, sending an e-mail to a physicist, etc.

The above-examples for the elastic net and SVM models have been given for the four VOC of interest. Here are the values of coefficients for using the elastic net test with the 65 VOC of interest.

**Table 3:** Coefficients of the elastic net test for 65 VOC of interest

| m/z | coef |
|---|---|
| 26.9994 | 0 |
| 27.0105 | 0 |
| 27.0218 | 0 |
| 27.9951 | 0 |
| 29.0026 | 0 |
| 29.014 | -0.17099834952128 |
| 29.0375 | 0 |
| 30.9838 | -0.19272254930386 |
| 31.0035 | 0 |
| 31.018 | 0 |
| 31.0545 | -0.0868809541462363 |
| 33.0338 | 0 |
| 38.0341 | -0.373544816475641 |
| 39.0231 | 0 |
| 39.033 | 0 |
| 41.0035 | 0 |
| 41.0392 | 0 |
| 43.0553 | 0 |
| 43.9676 | 0 |
| 43.9907 | 0 |
| 44.9981 | 0 |
| 45.0342 | 0 |
| 47.0135 | 0 |
| 47.05 | 0 |
| 51.0446 | 0 |
| 52.9618 | 0 |
| 53.002 | 0 |
| 53.0393 | 0 |
| 55.0549 | -0.204060119119194 |
| 57.0346 | 0 |

(continued)

| m/z | coef |
|---|---|
| 57.0706 | 0 |
| 60.0531 | 0 |
| 61.0356 | 0 |
| 62.0299 | -0.172910672454371 |
| 63.0079 | -0.133793047603517 |
| 65.0606 | 0 |
| 66.0475 | 0 |
| 66.9591 | 0 |
| 67.055 | -0.0185542639450894 |
| 68.0625 | 0 |
| 69.0706 | 0 |
| 71.0501 | -0.29614511174346 |
| 71.0861 | 0 |
| 73.0656 | 0 |
| 75.045 | 0 |
| 77.0604 | 0 |
| 79.0553 | 0.1565639655472 |
| 81.019 | 0 |
| 81.0708 | 0 |
| 83.0866 | -0.264806002067097 |
| 85.1024 | 0 |
| 87.0815 | 0 |
| 89.061 | 0 |
| 91.0563 | 0 |
| 93.0378 | 0.0134534777254841 |
| 97.0667 | 0.142381904312499 |
| 99.0818 | 0.116216917765426 |
| 107.0865 | 0.0435199756862549 |
| 111.1192 | 0.139762358652873 |
| 115.1135 | 0 |
| 117.0928 | 0 |
| 135.0885 | 0.34647275629029 |
| 137.1346 | 0.0746880721623011 |
| 143.1451 | 0.548099225194985 |
| 179.1459 | 0 |

Here are the values of coefficients for using the SVM test with the 65 VOC of interest.

**Table 4:** Coefficients of the SVM test for 65 VOC of interest

| features | coef | |
|---|---|---|
| intercept | 1.78457393935063 | |
| 26.9994 | 0.0203164775583294 | |
| 27.0105 | -0.00646293739846489 | |
| 27.0218 | 0.0131373528739178 | |
| 27.9951 | 0.00396558638862555 | |
| 29.0026 | 0.0105578163957263 | |
| 29.014 | -0.0490358531227796 | |
| 29.0375 | 0.0122541721514019 | |
| 30.9838 | -0.0794211818615996 | |
| 31.0035 | -0.0371246281462103 | |
| 31.018 | -0.00301229548201719 | |
| 31.0545 | -0.099084453254533 | |
| 33.0338 | -0.00907141264166196 | |
| 38.0341 | -0.029855704498521 | |
| 39.0231 | -0.0397990392900494 | |
| 39.033 | 0.00637568953724734 | |
| 41.0035 | -0.0230482514619407 | |
| 41.0392 | 0.011438473256849 | |
| 43.0553 | 0.0526010770568651 | |
| 43.9676 | -0.00876987827383436 | |
| 43.9907 | 0.0229029819998173 | |
| 44.9981 | 0.00858726062702962 | |
| 45.0342 | -0.00751150864277307 | |
| 47.0135 | 0.0140843908608015 | |
| 47.05 | -0.0152800023536971 | |
| 51.0446 | -0.0276965017027041 | |
| 52.9618 | -0.0294181148271273 | |
| 53.002 | -0.0114321026066049 | |
| 53.0393 | -0.0329220742342297 | |
| 55.0549 | -0.0796086805043102 | |
| 57.0346 | -0.0156023981925246 | |
| 57.0706 | 0.0165741125645432 | |
| 60.0531 | 0.0169707074015356 | |
| 61.0356 | 0.0476857892192711 | |
| 62.0299 | 0.00128519033348025 | |
| 63.0079 | -0.0347890132637384 | |
| 65.0606 | -0.00565899268637548 | |
| 66.0475 | -0.0159928201806251 | |

(continued)

| features | coef | |
|---|---|---|
| 66.9591 | -0.00725756349009642 | |
| 67.055 | -0.0442809159240086 | |
| 68.0625 | -0.0224115129980686 | |
| 69.0706 | -0.0183969983383 | |
| 71.0501 | -0.074340950426599 | |
| 71.0861 | 0.0085464014404322 | |
| 73.0656 | -0.0358032773974026 | |
| 75.045 | 0.00282342778864358 | |
| 77.0604 | -0.0216485052925101 | |
| 79.0553 | 0.0323336124905708 | |
| 81.019 | -0.0225294351687507 | |
| 81.0708 | 0.0121522697786908 | |
| 83.0866 | -0.0769266668049326 | |
| 85.1024 | 0.012202175586782 | |
| 87.0815 | -0.00812314515499904 | |
| 89.061 | 0.0196373308932 | |
| 91.0563 | 0.00999750102871999 | |
| 93.0378 | 0.040514992832867 | |
| 97.0667 | 0.0308882660672048 | |
| 99.0818 | 0.080845663301971 | |
| 107.0865 | 0.0264354320284494 | |
| 111.1192 | 0.0274064508385647 | |
| 115.1135 | 0.0518974143614893 | |
| 117.0928 | 0.0379433650316464 | |
| 135.0885 | 0.0901668878172821 | |
| 137.1346 | 0.028631519713326 | |
| 143.1451 | 0.0981198699268599 | |
| 179.1459 | -0.0135128151592857 | |

Thus, this method uses real-time, online, proton transfer reaction time-of-flight mass spectrometry to perform a metabolomic analysis of expired air from adults undergoing invasive mechanical ventilation in an intensive care unit due to severe COVID-19 or non-COVID-19 acute respiratory distress syndrome (ARDS). It is a real-time, non-invasive detection in exhaled air for identifying patients with COVID-19.

The invention is not limited to the above-described embodiments and many modifications of these embodiments could be made without departing from the invention.

Other types of spectrometer may be used. But this kind of spectrometer can directly receive expired air and provide immediately the result of the analysis. Other kinds of spectrometer may need more operations and deliver the result after a certain period of time.

For a given M molecule, the m/z values indicated above are characteristic of the mass spectrometer we used (PTR-TOF-MS). With this type of instrument, the molecule M whose mass would be equal to 200 is detected in its form [M+H]+ (the spectrometer "grafts" a proton to the molecule in order to give it an electric charge making detection possible), i.e. m/z = 201. It would also be possible to detect the same molecule M with other types of mass spectrometers based on different technologies, for example GC-MS, and in this case for this same molecule M, we would obtain masses corresponding to 4 or 5 fragments, for example 72, 125, 140 and 172. Still other types of mass spectrometers could also give values strictly identical to ours (SESI-MS...).

Examples include gas chromatography mass spectrometry (GCMS), liquid chromatography mass spectrometry (LCMS) and proton transfer reaction mass spectrometry (PTR-MS)), ion mobility spectrometry, field asymmetric ion mobility spectrometry, differential mobility spectrometry (DMS); infrared spectroscopy (IR spectroscopy) such as near-infrared (NIR), selected ion flow tube mass spectrometry (SIFT), Fourier Transform-Infrared (FOR) spectroscopy and ring-down, cavity spectroscopy or light absorption spectroscopy.

Mass spectrometry can be used it in tandem with chromatographic separation techniques. For example. GCMS is a analytical method that combines the features of gas chromatography and mass spectrometry to identify compounds. Similarly, liquid chromatography mass spectrometry (LCMS) is an analytical method that combines the features of liquid chromatography and mass spectrometry to identify compounds.

Ion-mobility spectrometry (IMS) is an analytical technique used to separate and identify ionized molecules in the gas phase based on their mobility in a carrier buffer gas. This technique can be coupled with mass spectrometry and/or chromatographic separation techniques. Having the person inspire pure oxygen is not compulsory. Other percentages of FiO2 may be used.

This device may be used on a person undergoing invasive mechanical ventilation, but it may also be used on a person who is not undergoing that. It could be a person who is sick or suspected to be sick and/or who has been diagnosed as affected by the COVID-19. It could be a person having ARDS or no ARDS. It could be a person having symptoms or no symptoms. For persons not undergoing mechanical ventilation, sampling devices other than the transfer line directly connected to the end of the endotracheal tube may be used, such as the Buffered End-Tidal Breath Sampling Inlet (BET-med device, Ionicon, Innsbruck).

The number of VOC (or ranges of m/z) of interest has been illustrated with examples of 4, 8 and 65. But many other numbers are possible, for example 10, 12, 20, 30, etc., provided a test or decision rule is properly designed to be used with this number of VOC.

Besides, when using a number of VOC less than 65, the chosen VOC (or ranges of m/z) of interest need not be among the most prominent ones in the complete signature of 65. A good signature may be obtained with a number of VOC (and an associated test or decision rule) not among the most prominent ones.

**Claims**

1. A method for analysis, the method comprising:

    - obtaining a sample comprising elements coming from an air expired from a person (112);
    - exposing the sample to a spectrometer;
    - determining values of signal intensities and/or concentrations of ions defined by their mass-to-charge ratios (m/z) given by the spectrometer in a group of ranges,

    each range being defined by a median value and limits of the range;

    - applying at least one test to the values,

    the group and the test being configured for identifying a patient suffering from COVID-19; and

    - communicating a message according to a result of the test.

2. A method according to the preceding claim wherein the spectrometer is a mass spectrometer, for example a proton transfer reaction mass spectrometer.

3. A method according to claim 2, wherein the group comprises at least one of the following ranges, preferably all of them, and for example consists in all of them:

    - m/z = 135.09 (135.03-135.17),
    - m/z = 143.15 (143.07-143.22),
    - m/z = 99.08 (99.04-99.14), and
    - m/z = 111.12 (111.07-111.18).

4. A method according to claim 2, wherein the group comprises at least one of the following ranges, preferably all of them, and for example consists in all of them:

- m/z = 135.09 (135.03-135.17),
- m/z = 143.15 (143.07-143.22),
- m/z = 99.08 (99.04-99.14),
- m/z = 111.12 (111.07-111.18),
- m/z = 71.05 (71.02-71.09),
- m/z = 55.05 (55.03-55.09),
- m/z = 83.09 (83.05-83.13), and
- m/z = 93.04 (93.00-93.09).

5. A method according to claim 2, wherein the group comprises at least one of the ranges of the following table, preferably all of them, and for example consists in all of them:

| (m/z [M+H]$^+$) median | (m/z [M+H]$^+$) lower limit | (m/z [M+H]$^+$) upper limit |
|---|---|---|
| 135,09 | 135,03 | 135,17 |
| 143,15 | 143,07 | 143,22 |
| 99,08 | 99,04 | 99,14 |
| 111,12 | 111,07 | 111,18 |
| 71,05 | 71,02 | 71,08 |
| 55,05 | 55,03 | 55,09 |
| 83,09 | 83,05 | 83,13 |
| 93,04 | 93,00 | 93,09 |
| 29,01 | 29,00 | 29,03 |
| 115,11 | 115,05 | 115,18 |
| 97,07 | 97,02 | 97,10 |
| 63,01 | 62,98 | 63,04 |
| 47,05 | 47,03 | 47,08 |
| 65,06 | 65,03 | 65,10 |
| 179,15 | 179,07 | 179,25 |
| 73,07 | 73,04 | 73,11 |
| 30,98 | 30,97 | 31,00 |
| 79,06 | 79,02 | 79,10 |
| 81,07 | 81,04 | 81,12 |
| 38,03 | 38,02 | 38,06 |
| 39,02 | 39,01 | 39,04 |
| 53,04 | 53,02 | 53,07 |
| 85,10 | 85,07 | 85,15 |
| 81,02 | 80,98 | 81,06 |
| 41,00 | 40,98 | 41,03 |
| 117,09 | 117,04 | 117,16 |
| 43,99 | 43,97 | 44,02 |
| 31,00 | 30,99 | 31,02 |

(continued)

| (m/z [M+H]$^+$) median | (m/z [M+H]$^+$) lower limit | (m/z [M+H]$^+$) upper limit |
|---|---|---|
| 71,09 | 71,06 | 71,13 |
| 45,03 | 45,01 | 45,06 |
| 43,97 | 43,94 | 43,99 |
| 89,06 | 89,02 | 89,11 |
| 66,96 | 66,93 | 67,00 |
| 107,09 | 107,03 | 107,15 |
| 91,06 | 91,02 | 91,11 |
| 53,00 | 52,97 | 53,03 |
| 75,05 | 75,01 | 75,09 |
| 27,00 | 26,98 | 27,01 |
| 31,02 | 31,00 | 31,03 |
| 67,06 | 67,03 | 67,09 |
| 60,05 | 60,03 | 60,09 |
| 45,00 | 44,98 | 45,02 |
| 39,03 | 39,01 | 39,05 |
| 27,01 | 26,99 | 27,03 |
| 33,03 | 33,02 | 33,05 |
| 41,04 | 41,02 | 41,06 |
| 29,00 | 28,99 | 29,02 |
| 77,06 | 77,03 | 77,10 |
| 31,05 | 31,04 | 31,07 |
| 61,04 | 61,00 | 61,07 |
| 28,00 | 27,98 | 28,01 |
| 57,03 | 57,01 | 57,06 |
| 137,13 | 137,07 | 137,21 |
| 57,07 | 57,05 | 57,10 |
| 62,03 | 62,00 | 62,06 |
| 68,06 | 68,04 | 68,10 |
| 27,02 | 27,01 | 27,04 |
| 43,06 | 43,04 | 43,08 |
| 87,08 | 87,04 | 87,13 |
| 47,01 | 46,99 | 47,04 |
| 52,96 | 52,94 | 52,99 |
| 66,05 | 66,02 | 66,08 |
| 51,04 | 51,02 | 51,07 |
| 69,07 | 69,05 | 69,11 |
| 29,04 | 29,02 | 29,05 |

**6.** The method of any of the preceding claims wherein the person (112) is undergoing invasive mechanical ventilation.

**7.** The method of any of the preceding claims wherein the sample is a sample of the expired air.

**8.** The method of any of the preceding claims wherein the step of obtaining a sample comprises obtaining the sample via a transfer line (106) in direct communication with the person (112), for example the transfer line (106) being connected to an end of an endotracheal tube (110) installed on the person (112).

**9.** The method of the preceding claim, comprising heating the transfer line (106).

**10.** A device (120) for the diagnosis, screening or monitoring of COVID-19,
the device comprising:

- means for determining values of signal intensities and/or concentrations of ions defined by their mass-to-charge ratios (m/z) given by a spectrometer in a group of ranges,

each range being defined by a median value and limits of the range; and

- means for applying a predetermined test to the intensities;

the group and the test being configured for identifying a patient suffering from COVID-19.

**11.** The device of the preceding claim, comprising a spectrometer, such as a mass spectrometer, for example a proton-transfer-reaction mass spectrometer (118).

**12.** A program comprising code instructions configured for controlling an execution of a method according to claims 1 to 9 or for controlling a device (120; 220) according to claims 10 to 11.

**13.** A process for providing a program according to the preceding claim on a communication network in view of down-loading the program.

EP 3 971 569 A1

# FIG. 1

PCA

OPLS-DA

COVID-19 disease

## FIG. 2

| Model | Accuracy | Sensitivity | Specificity | AUC | Number of features |
|---|---|---|---|---|---|
| ElasticNet | 0.93 | 0.9 | 0.94 | 0.93 | 19 |
| Random Forest | 0.93 | 0.9 | 0.94 | 0.98 | 16 |
| SVM | 0.93 | 0.9 | 0.94 | 0.96 | 65 |

## FIG. 2 (continued)

| Model | Accuracy | Sensitivity | Specificity | AUC | Dimension |
|---|---|---|---|---|---|
| ElasticNet | 0.93 | 0.90 | 0.94 | 0.97 | 8 |
| Random Forest | 0.90 | 0.85 | 0.93 | 0.98 | 8 |
| SVM | 0.92 | 0.90 | 0.93 | 0.95 | 8 |

EP 3 971 569 A1

# FIG. 2 (continued)

| Model | Accuracy | Sensitivity | Specificity | AUC | Number of features |
|---|---|---|---|---|---|
| ElasticNet | 0.93 | 0.90 | 0.94 | 0.95 | 4 |
| Random Forest | 0.91 | 0.98 | 0.88 | 0.89 | 4 |
| SVM | 0.89 | 0.90 | 0.89 | 0.94 | 4 |

EP 3 971 569 A1

FIG. 3

FIG. 3 (continued)

**FIG. 4**

Tidal volume

p-value = 0.55

dim2 (18%)

dim1 (34%)

400 500 600

CRP

p-value = 0.07

dim2 (18%)

dim1 (34%)

100 200 300 400 500

Body temperature

p-value = 0.55

dim2 (18%)

dim1 (34%)

35 36 37 38

## FIG. 5

### First acquisition available per patient

*m/z* 99.0818

*m/z* 135.0885

*m/z* 143.1451

*m/z* 111.1192

# FIG. 5 (continued)

## Longitudinal modeling

### *m/z* 99.0818

### *m/z* 135.0885

COVID-19 disease ⋯⋯ −
___ +

# FIG. 5 (continued)

## Longitudinal modeling

### 143.1451 *m/z*

### *m/z* 111.1192

COVID-19 disease

FIG. 6

FIG. 6 (continued)

111.118

135.083

EP 3 971 569 A1

FIG. 6 (continued)

99.0815

145.1235

111.1191

135.0986

EP 3 971 569 A1

EP 3 971 569 A1

**FIG. 7**

FIG. 8

EP 3 971 569 A1

FIG. 9

EP 3 971 569 A1

# FIG. 10

X135.0885< -1.416

X99.0818>=-0.7923

0          1

1

Europäisches Patentamt

European Patent Office

Office européen des brevets

# EUROPEAN SEARCH REPORT

**Application Number**

EP 20 30 6170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GOULD OLIVER ET AL: "Breath analysis for detection of viral infection, the current position of the field", JOURNAL OF BREATH RESEARCH, vol. 14, no. 4, 21 July 2020 (2020-07-21), page 041001, XP055783242, DOI: 10.1088/1752-7163/ab9c32 * abstract; discussion * | 1-13 | INV. G01N33/68 G01N33/497 G01N33/569 |
| Y,P | WO 2020/186335 A1 (CANARY HEALTH TECH INC [CA]) 24 September 2020 (2020-09-24) * par. 76, 87, par. 197-199 * | 1-13 | |
| A,D | LIEUWE DJ BOS ET AL: "Exhaled breath profiling for diagnosing acute respiratory distress syndrome", BMC PULMONARY MEDICINE, BIOMED CENTRAL, LONDON, GB, vol. 14, no. 1, 26 April 2014 (2014-04-26), page 72, XP021181771, ISSN: 1471-2466, DOI: 10.1186/1471-2466-14-72 * the whole document * | 1-13 | |
| Y,D | SCHUBERT J K ET AL: "Application of a new method for analysis of exhaled gas in critically ill patients", INTENSIVE CARE MEDICINE, SPRINGER BERLIN HEIDELBERG, BERLIN/HEIDELBERG, vol. 24, no. 5, 1 May 1998 (1998-05-01), pages 415-421, XP037118966, ISSN: 0342-4642, DOI: 10.1007/S001340050589 [retrieved on 2014-02-26] * abstract, "gas chromatographic analysis" * | 1-13 | |

**TECHNICAL FIELDS SEARCHED (IPC)**

G01N

The present search report has been drawn up for all claims

| Place of search | Date of completion of the search | Examiner |
|---|---|---|
| Munich | 9 March 2021 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 6170

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2021

| Patent document cited in search report | Publication date | Patent family member(s) | Publication date |
|---|---|---|---|
| WO 2020186335 A1 | 24-09-2020 | US 2020337594 A1<br>WO 2020186335 A1 | 29-10-2020<br>24-09-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- COVID-19 Dashboard by the Center for Systems Science and Engineering (CSSE). Johns Hopkins University (JHU), 28 September 2020
- **WIERSINGA WJ ; RHODES A ; CHENG AC ; PEACOCK SJ ; PRESCOTT HC.** Pathophysiology, Transmission, Diagnosis, and Treatment of Coronavirus Disease 2019 (COVID-19): A Review. *JAMA,* 2020
- **BEIGEL JH ; TOMASHEK KM ; DODD LE et al.** Remdesivir for the Treatment of Covid-19 - Preliminary Report. *N Engl J Med,* 2020
- **RECOVERY_COLLABORATIVE_GROUP ; HORBY P ; LIM WS et al.** Dexamethasone in Hospitalized Patients with Covid-19 - Preliminary Report. *N Engl J Med,* 2020
- **LUCAS C ; WONG P ; KLEIN J et al.** Longitudinal analyses reveal immunological misfiring in severe COVID-19. *Nature,* 2020
- **HADJADJ J ; YATIM N ; BARNABEI L et al.** Impaired type I interferon activity and inflammatory responses in severe COVID-19 patients. *Science,* 2020
- **KURI-CERVANTES L ; PAMPENA MB ; MENG W et al.** Comprehensive mapping of immune perturbations associated with severe COVID-19. *Sci Immunol,* 2020, vol. 5 (49
- **SHEN B ; YI X ; SUN Y et al.** Proteomic and Metabolomic Characterization of COVID-19 Patient Sera. *Cell,* 2020, vol. 182 (1), 59-72 e15
- **KATAOKA H ; SAITO K ; KATO H ; MASUDA K.** Noninvasive analysis of volatile biomarkers in human emanations for health and early disease diagnosis. *Bioanalysis,* 2013, vol. 5 (11), 1443-59
- **RATTRAY NJ ; HAMRANG Z ; TRIVEDI DK ; GOODACRE R ; FOWLER SJ.** Taking your breath away: metabolomics breathes life in to personalized medicine. *Trends Biotechnol,* 2014, vol. 32 (10), 538-48
- **AMANN A ; DE LACY COSTELLO B ; MIEKISCH W et al.** The human volatilome: volatile organic compounds (VOCs) in exhaled breath, skin emanations, urine, feces and saliva. *J Breath Res,* 2014, vol. 8 (3), 034001
- **DE LACY COSTELLO B ; AMANN A ; AL-KATEB H et al.** A review of the volatiles from the healthy human body. *J Breath Res,* 2014, vol. 8 (1), 014001

- **KOO S ; THOMAS HR ; DANIELS SD et al.** A breath fungal secondary metabolite signature to diagnose invasive aspergillosis. *Clin Infect Dis,* 2014, vol. 59 (12), 1733-40
- **NAKHLEH MK ; JERIES R ; GHARRA A et al.** Detecting active pulmonary tuberculosis with a breath test using nanomaterial-based sensors. *Eur Respir J,* 2014, vol. 43 (5), 1522-5
- **CORONEL TEIXEIRA R ; RODRIGUEZ M ; JIMENEZ DE ROMERO N et al.** The potential of a portable, point-of-care electronic nose to diagnose tuberculosis. *J Infect,* 2017, vol. 75 (5), 441-7
- **SUAREZ-CUARTIN G ; GINER J ; MERINO JL et al.** Identification of Pseudomonas aeruginosa and airway bacterial colonization by an electronic nose in bronchiectasis. *Respir Med,* 2018, vol. 136, 111-7
- **SCHNABEL R ; FIJTEN R ; SMOLINSKA A et al.** Analysis of volatile organic compounds in exhaled breath to diagnose ventilator-associated pneumonia. *Sci Rep,* 2015, vol. 5, 17179
- **FILIPIAK W ; BEER R ; SPONRING A et al.** Breath analysis for in vivo detection of pathogens related to ventilator-associated pneumonia in intensive care patients: a prospective pilot study. *J Breath Res,* 2015, vol. 9 (1), 016004
- **SCHNABEL RM ; BOUMANS ML ; SMOLINSKA A et al.** Electronic nose analysis of exhaled breath to diagnose ventilator-associated pneumonia. *Respir Med,* 2015, vol. 109 (11), 1454-9
- **BOS LD ; SCHULTZ MJ ; STERK PJ.** Exhaled breath profiling for diagnosing acute respiratory distress syndrome. *BMC Pulm Med,* 2014, vol. 14, 72
- **BOS LD ; WEDA H ; WANG Y et al.** Exhaled breath metabolomics as a noninvasive diagnostic tool for acute respiratory distress syndrome. *Eur Respir J,* 2014, vol. 44 (1), 188-97
- **SCHUBERT JK ; MULLER WP ; BENZING A ; GEIGER K.** Application of a new method for analysis of exhaled gas in critically ill patients. *Intensive Care Med,* 1998, vol. 24 (5), 415-21
- **VAN GEFFEN WH ; BRUINS M ; KERSTJENS HA.** Diagnosing viral and bacterial respiratory infections in acute COPD exacerbations by an electronic nose: a pilot study. *J Breath Res,* 2016, vol. 10 (3), 036001

- **TRAXLER S ; BISCHOFF AC ; SASS R et al.** VOC breath profile in spontaneously breathing awake swine during Influenza A infection. *Sci Rep,* 2018, vol. 8 (1), 14857
- **ACKERMANN M ; VERLEDEN SE ; KUEHNEL M et al.** Pulmonary Vascular Endothelialitis, Thrombosis, and Angiogenesis in Covid-19. *N Engl J Med,* 2020, vol. 383 (2), 120-8
- **FORCE ADT ; RANIERI VM ; RUBENFELD GD et al.** Acute respiratory distress syndrome: the Berlin Definition. *JAMA,* 2012, vol. 307 (23), 2526-33
- **LE GALL JR ; LEMESHOW S ; SAULNIER F.** A new Simplified Acute Physiology Score (SAPS II) based on a European/North American multicenter study. *JAMA,* 1993, vol. 270 (24), 2957-63
- **VINCENT JL ; MORENO R ; TAKALA J et al.** The SOFA (Sepsis-related Organ Failure Assessment) score to describe organ dysfunction/failure. On behalf of the Working Group on Sepsis-Related Problems of the European Society of Intensive Care Medicine. *Intensive Care Med,* 1996, vol. 22 (7), 707-10
- **TREFZ P ; PUGLIESE G ; BROCK B ; SCHUBERT JK ; MIEKISCH W.** Effects of elevated oxygen levels on VOC analysis by means of PTR-ToF-MS. *J Breath Res,* 2019, vol. 13 (4), 046004
- **BENJAMINI Y ; HOCHBERG Y.** Controlling the False Discovery Rate: A Practical and Powerful Approach to Multiple Testing. *J R Stat Soc Ser B,* 1995, vol. 57 (1), 289-300
- **THEVENOT EA ; ROUX A ; XU Y ; EZAN E ; JUNOT C.** Analysis of the Human Adult Urinary Metabolome Variations with Age, Body Mass Index, and Gender by Implementing a Comprehensive Workflow for Univariate and OPLS Statistical Analyses. *J Proteome Res,* 2015, vol. 14 (8), 3322-35
- **ZOU H ; HASTIE T.** Regularization and variable selection via the elastic net. *J R Stat Soc Ser B,* 2005, vol. 67 (2), 301-20
- **BREIMAN L ; RANDOM FORESTS.** *Machine Learning,* 2001, vol. 45 (1), 5-32
- **WESTON J ; MUKHERJEE S ; CHAPELLE O ; PONTIL M ; POGGIO T ; VAPNIK V.** Feature Selection for SVMs. *Advances in Neural Information Processing Systems,* 2000, vol. 13
- **PIHUR V ; DATTA S ; DATTA S.** RankAggreg, an R package for weighted rank aggregation. *BMC Bioinformatics,* 2009, vol. 10, 62
- **TREFZ P ; SCHMIDT M ; OERTEL P et al.** Continuous real time breath gas monitoring in the clinical environment by proton-transfer-reaction-time-of-flight-mass spectrometry. *Anal Chem,* 2013, vol. 85 (21), 10321-9
- **BROCK B ; KAMYSEK S ; SILZ J ; TREFZ P ; SCHUBERT JK ; MIEKISCH W.** Monitoring of breath VOCs and electrical impedance tomography under pulmonary recruitment in mechanically ventilated patients. *J Breath Res,* 2017, vol. 11 (1), 016005
- **VAN DE KANT KD ; VAN BERKEL JJ ; JOBSIS Q et al.** Exhaled breath profiling in diagnosing wheezy preschool children. *Eur Respir J,* 2013, vol. 41 (1), 183-8
- **CORRADI M ; PIGNATTI P ; MANINI P et al.** Comparison between exhaled and sputum oxidative stress biomarkers in chronic airway inflammation. *Eur Respir J,* 2004, vol. 24 (6), 1011-7
- **RAHMAN I.** Oxidative stress, chromatin remodeling and gene transcription in inflammation and chronic lung diseases. *J Biochem Mol Biol,* 2003, vol. 36 (1), 95-109